# EUROPEAN PATENT APPLICATION

(11) **EP 0 781 847 A1**
(43) Date of publication of application: **02.07.1997**
(21) Application number: 96117154.3
(22) Date of filing: 25.10.1996
(51) Int. Cl.: C12N 15/62, C12N 15/13, C07K 16/46, C07K 16/30, C12N 5/10, A61K 39/395

(54) **Humanized monoclonal antibody**

(30) Priority: 06.11.1995 EP 95117407
(71) Applicant: MERCK PATENT GmbH, 64293 Darmstadt (DE)
(72) Inventor: Bendig, Mary, Dr., London N200XL (GB); Saldana, Jose, Dr., Enfield MDDx, EN1 1TE (GB); Jones, Tarran, Dr., Cerscent Radlett, Hartfordshire WD7 BAG (GB)

(57) **Abstract**

The invention relates to a new humanized and reshaped monoclonal antibody which derives from murine monclonal antibody 15 (MAb 15), DNA and amino acid sequences and pharmaceutical compositions comprising this antibody or fragments thereof for the use of treating tumors, especially lung cancer.

SEQUENCE LISTING

(1) GENERAL INFORMATION:

(i) APPLICANT:
(A) NAME: Merck Patent GmbH
(B) STREET: Frankfurter Str. 250
(C) CITY: Darmstadt
(E) COUNTRY: Germany
(F) POSTAL CODE (ZIP): 64293
(G) TELEPHONE: 49-6151-72 7022
(H) TELEFAX: 49-6151-72 6191

(ii) TITLE OF INVENTION: Humanized Monoclonal Antibody

(iii) NUMBER OF SEQUENCES: 55

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96 117 154.3

(2) INFORMATION FOR SEQ ID NO: 1:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..41
(D) OTHER INFORMATION:/function= "Light chain primer
(41mer), leader"
/product= "MAb 15 variable region"

(xi) SEQUENCE DESCRIPTION: SEQ ID NO: 1:

ACTAGTCGAC ATGGGCATCA AGATGGAGTC ACAGACCCAG G 41

(2) INFORMATION FOR SEQ ID NO: 2:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..41
(D) OTHER INFORMATION:/function= "Light chain primer
(41mer, modification)"
/product= "MAb 15, variable region"

(xi) SEQUENCE DESCRIPTION: SEQ ID NO: 2:

ACTAGTCGAC ATGGGCTTCA AGATGGAGTC ACATTTTCTG G 41

(2) INFORMATION FOR SEQ ID NO: 3:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..27
(D) OTHER INFORMATION:/function= "Kappa constant region
primer (27mer), light chain"
/product= "MAb 15, variable region"

(xi) SEQUENCE DESCRIPTION: SEQ ID NO: 3:

GGATCCCGGG TGGATGGTGG GAAGATG 27

(2) INFORMATION FOR SEQ ID NO: 4:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..35
(D) OTHER INFORMATION:/function= "Heavy chain ptimer
(35mer), leader sequence"
/product= "MAb 15, variable region"

(xi) SEQUENCE DESCRIPTION: SEQ ID NO: 4:

ACTAGTCGAC ATGAACTTTG GGCTCAGCTT GATTT 35

(2) INFORMATION FOR SEQ ID NO: 5:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..35
(D) OTHER INFORMATION:/function= "Heavy chain primer,
leader (35mer)"
/product= "MAb 15, variable region"

(xi) SEQUENCE DESCRIPTION: SEQ ID NO: 5:

ACTAGTCGAC ATGGACTTTG GGTTCAGCTT GGTTT 35

(2) INFORMATION FOR SEQ ID NO: 6:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..28
(D) OTHER INFORMATION:/function= "Heavy chain primer
(28mer), Gamma-1 constant region reverse"
/product= "MAb 15, variable region"

(xi) SEQUENCE DESCRIPTION: SEQ ID NO: 6:

GGATCCCGGG CCAGTGGATA GACAGATG 28

(2) INFORMATION FOR SEQ ID NO: 7:

(i) SEQUENCE CHARACTERISTICS:
(A) LENGTH: 37 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..19
(D) OTHER INFORMATION:/function= "Primer(37mer) light
chain, Kozak sequence + HindIII"
/product= "Chimeric MAb 15, variable region"

(ix) FEATURE:
(A) NAME/KEY: CDS
(B) LOCATION:20..37
(D) OTHER INFORMATION:/function= "Primer(37mer), coding
region"
/product= "MAb 15 chimeric, variable region"

(xi) SEQUENCE DESCRIPTION: SEQ ID NO: 7:

ATATAAGCTT GCCGCCACC ATG GGC ATC AAG ATG GAG 37
Met Gly Ile Lys Met Glu
1               5

(2) INFORMATION FOR SEQ ID NO: 8:

(i) SEQUENCE CHARACTERISTICS:
(A) LENGTH: 6 amino acids
(B) TYPE: amino acid
(D) TOPOLOGY: linear

(ii) MOLECULE TYPE: protein
(xi) SEQUENCE DESCRIPTION: SEQ ID NO: 8:

Met Gly Ile Lys Met Glu
1               5

(2) INFORMATION FOR SEQ ID NO: 9:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..35
(D) OTHER INFORMATION:/function= "light chain primer
(35mer), BamHI, Splice donor site"
/product= "Chimeric MAb 15, variable region"

(xi) SEQUENCE DESCRIPTION: SEQ ID NO: 9:

TATAGGATCC ACTCACGTTT GATTTCCAGC TTGGT 35

(2) INFORMATION FOR SEQ ID NO: 10:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..15
(D) OTHER INFORMATION:/function= "Heavy chain primer
(33mer), HindIII + Kozak sequence"
/product= "Chimeric MAb15, variable region"

(ix) FEATURE:
(A) NAME/KEY: CDS
(B) LOCATION:16..33
(D) OTHER INFORMATION:/function= "Heavy chain primer,
coding sequence"
/product= "Chimeric MAb15, variable region"

(xi) SEQUENCE DESCRIPTION: SEQ ID NO: 10:

ATCGAAGCTT CCACC ATG AAC TTT GGG CTC AGC 33
Met Asn Phe Gly Leu Ser
10

(2) INFORMATION FOR SEQ ID NO: 11:

(i) SEQUENCE CHARACTERISTICS:
(A) LENGTH: 6 amino acids
(B) TYPE: amino acid
(D) TOPOLOGY: linear

(ii) MOLECULE TYPE: protein
(xi) SEQUENCE DESCRIPTION: SEQ ID NO: 11:

Met Asn Phe Gly Leu Ser
1               5

(2) INFORMATION FOR SEQ ID NO: 12:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..35
(D) OTHER INFORMATION:/function= "Heavy chain primer,
BamH1, splice donor site"
/product= "Chimeric MAb 15, variable region"

(xi) SEQUENCE DESCRIPTION: SEQ ID NO: 12:

ATTAGGATCC ACTCACCTGC AGAGACAGTG ACCAG 35

(2) INFORMATION FOR SEQ ID NO: 13:

(i) SEQUENCE CHARACTERISTICS:
(A) LENGTH: 53 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: CDS
(B) LOCATION:1..51
(D) OTHER INFORMATION:/function= "light chain primer
(53mer) CDR1 front PCRprimer"
/product= "Reshaped MAb 15, variable region"

(xi) SEQUENCE DESCRIPTION: SEQ ID NO: 13:

CTT TTA TAT AGT AGC AAT CAA AAG AAC TAC TTG GCC TGG TAC CAG CAG 48
Leu Leu Tyr Ser Ser Asn Gln Lys Asn Tyr Leu Ala Trp Tyr Gln Gln
10 15 20

AAG CC 53
Lys

(2) INFORMATION FOR SEQ ID NO: 14:

(i) SEQUENCE CHARACTERISTICS:
(A) LENGTH: 17 amino acids
(B) TYPE: amino acid
(D) TOPOLOGY: linear

(ii) MOLECULE TYPE: protein
(xi) SEQUENCE DESCRIPTION: SEQ ID NO: 14:

Leu Leu Tyr Ser Ser Asn Gln Lys Asn Tyr Leu Ala Trp Tyr Gln Gln
1               5                   10                  15

Lys

(2) INFORMATION FOR SEQ ID NO: 15:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..51
(D) OTHER INFORMATION:/function= "light chain
primer(51mer), CDR1 back PCR primer"
/product= "Reshaped MAb 15, variable region"

(xi) SEQUENCE DESCRIPTION: SEQ ID NO: 15:

GATTGCTACT ATATAAAAGG CTCTGACTGG ACTTACAGGT GATGGTCACT C 51

(2) INFORMATION FOR SEQ ID NO: 16:

(i) SEQUENCE CHARACTERISTICS:
(A) LENGTH: 59 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: CDS
(B) LOCATION:1..57
(D) OTHER INFORMATION:/function= "light chain
primer(59mer), CDR2"

/product= "Reshaped MAb 15, variable region"

(xi) SEQUENCE DESCRIPTION: SEQ ID NO: 16:

AAG CTG CTG ATC TAC TGG GCA TCC ACT AGG GAA TCT GGT GTG CAA GAC 48
Lys Leu Leu Ile Tyr Trp Ala Ser Thr Arg Glu Ser Gly Val Gln Asp
20 25 30

AGA TTC AGC GG 59
Arg Phe Ser
35

(2) INFORMATION FOR SEQ ID NO: 17:

(i) SEQUENCE CHARACTERISTICS:
(A) LENGTH: 19 amino acids
(B) TYPE: amino acid
(D) TOPOLOGY: linear

(ii) MOLECULE TYPE: protein
(xi) SEQUENCE DESCRIPTION: SEQ ID NO: 17:

Lys Leu Leu Ile Tyr Trp Ala Ser Thr Arg Glu Ser Gly Val Gln Asp
1               5                   10                  15

Arg Phe Ser

(2) INFORMATION FOR SEQ ID NO: 18:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: CDS
(B) LOCATION:1..51
(D) OTHER INFORMATION:/function= "light chain
primer(51mer), CDR3"
/product= "Reshaped MAb 15, variable region"

(xi) SEQUENCE DESCRIPTION: SEQ ID NO: 18:

ACC TAC TAC TGC CAG CAA TAT TCT AAC TAT CCT CCG ACG TTC GGC CAA 48
Thr Tyr Tyr Cys Gln Gln Tyr Ser Asn Tyr Pro Pro Thr Phe Gly Gln
20 25 30 35

GGG 51
Gly

(2) INFORMATION FOR SEQ ID NO: 19:

(i) SEQUENCE CHARACTERISTICS:
(A) LENGTH: 17 amino acids
(B) TYPE: amino acid
(D) TOPOLOGY: linear

(ii) MOLECULE TYPE: protein
(xi) SEQUENCE DESCRIPTION: SEQ ID NO: 19:

Thr Tyr Tyr Cys Gln Gln Tyr Ser Asn Tyr Pro Pro Thr Phe Gly Gln
1               5                   10                  15

Gly

(2) INFORMATION FOR SEQ ID NO: 20:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..18
(D) OTHER INFORMATION:/function= "forward primer
APCR4(18mer)"
/product= "Reshaped MAb 15, variable region"

(xi) SEQUENCE DESCRIPTION: SEQ ID NO: 20:

GAGTGCACCA TATGCGGT 18

(2) INFORMATION FOR SEQ ID NO: 21:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..17
(D) OTHER INFORMATION:/function= "forward primer
UP(17mer)"
/product= "reshaped MAb15, variable region"

(xi) SEQUENCE DESCRIPTION: SEQ ID NO: 21:

GATAAACGAC GGCCAGT 17

(2) INFORMATION FOR SEQ ID NO: 22:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..17
(D) OTHER INFORMATION:/function= "backward primer
APCR1(17mer)"
/product= "Reshaped MAb 15, variable region"

(xi) SEQUENCE DESCRIPTION: SEQ ID NO: 22:

TACGCAAACC GCCTCTC 17

(2) INFORMATION FOR SEQ ID NO: 23:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..16
(D) OTHER INFORMATION:/function= "backward primer RSP
(16mer)"
/product= "Reshaped MAb15, variable region"

(xi) SEQUENCE DESCRIPTION: SEQ ID NO: 23:

AACAGCTATG ACCATG 16

(2) INFORMATION FOR SEQ ID NO: 24:

(i) SEQUENCE CHARACTERISTICS:
(A) LENGTH: 88 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..88
(D) OTHER INFORMATION:/function= "forward PCR primer
(88mer), heavy chain"
/product= "Reshaped MAb 15 variable region,
version a"

(xi) SEQUENCE DESCRIPTION: SEQ ID NO: 24:

ATTAAAGCTT GCCGCCACCA TGGAGTTTGG GCTGAGCTGG CTTTTTCTTG TGGCTATTTT 60

AAAAGGTGTC CAGTGTGAGG TGCAGCTG 88

(2) INFORMATION FOR SEQ ID NO: 25:

(i) SEQUENCE CHARACTERISTICS:
(A) LENGTH: 89 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..89
(D) OTHER INFORMATION:/function= "backward PCR
primer(89mer), heavy chain"
/product= "Reshaped MAb15, variable region,
version A"

(xi) SEQUENCE DESCRIPTION: SEQ ID NO: 25:

GTGAATCCGG AGGCTGCACA GGAGAGTCTC AGGGACCCCC CAGGCTGTAC CAAGCCTCCC 60

CCAGACTCCA ACAGCTGCAC CTCACACTG 89

(2) INFORMATION FOR SEQ ID NO: 26:

(i) SEQUENCE CHARACTERISTICS:
(A) LENGTH: 88 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..88
(D) OTHER INFORMATION:/function= "forward PCR primer
(88mer), heavy chain"
/product= "Reshaped MAb 15, variable region,
version A"

(xi) SEQUENCE DESCRIPTION: SEQ ID NO: 26:

CAGCCTCCGG ATTCACCTTT AGCGACTATG ACATGTCTTG GGTCCGCCAG GCACCAGGGA 60

AGAGGCTGGA GTGGGTCGCA TACCTTAG 88

(2) INFORMATION FOR SEQ ID NO: 27:

(i) SEQUENCE CHARACTERISTICS:
(A) LENGTH: 85 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..85
(D) OTHER INFORMATION:/function= "backward PCR
primer(85mer), heavy chain"
/product= "Reshaped MAb 15 variable region,
version A"

(xi) SEQUENCE DESCRIPTION: SEQ ID NO: 27:

GAATTGTCTC TCGAGATGGT GAATCGGCCC TTCACAGTGT CTGGATAGTA GGTGCTACCA 60

CCACCTCTAC TAAGGTATGC GACCC 85

(2) INFORMATION FOR SEQ ID NO: 28:

(i) SEQUENCE CHARACTERISTICS:
(A) LENGTH: 96 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..96
(D) OTHER INFORMATION:/function= "forward PCR
primer(96mer), heavy chain"
/product= "Reshaped MAb 15, variable region,
version A"

(xi) SEQUENCE DESCRIPTION: SEQ ID NO: 28:

CCATCTCGAG AGACAATTCC AAGAACACGC TGTATCTGCA AATGAACAGC CTGAGAGCCG 60

AGGACACGGC CGTATATTAC TGTGCGAGAC ATGGTG 96

(2) INFORMATION FOR SEQ ID NO: 29:

(i) SEQUENCE CHARACTERISTICS:
(A) LENGTH: 93 base pairs

(B) TYPE: nucleic acid

(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..93
(D) OTHER INFORMATION:/function= "backward primer
(93mer), heavy chain"
/product= "Reshaped MAb 15, variable region,
version A"

(xi) SEQUENCE DESCRIPTION: SEQ ID NO: 29:

TATAGGATCC ACTCACCTGA GGAGACGGTG ACCAGGGTTC CCTGGCCCCA GTAATCAAAC 60

CAGGGGCGGA CCTCCTCACC ATGTCTCGCA CAG 93

(2) INFORMATION FOR SEQ ID NO: 30:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..22
(D) OTHER INFORMATION:/function= "Forward PCR primer
RH15.sense (22mer), heavy chain"
/product= "Reshaped MAb 15, variable region.
version A"

(xi) SEQUENCE DESCRIPTION: SEQ ID NO: 30:

ATTAAAGCTT GCCGCCACCA TG 22

(2) INFORMATION FOR SEQ ID NO: 31:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..19
(D) OTHER INFORMATION:/function= "backward PCR primer
RH15.antisense (19mer), heavy chain"
/product= "Reshaped MAb 15, variable region
version A"

(xi) SEQUENCE DESCRIPTION: SEQ ID NO: 31:

TATAGGATCC ACTCACCTG 19

(2) INFORMATION FOR SEQ ID NO: 32:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..30
(D) OTHER INFORMATION:/function= "forward PCR
primer(30mer), heavy chain"
/product= "Reshaped MAb 15, variable region,
version B"

(xi) SEQUENCE DESCRIPTION: SEQ ID NO: 32:

GCCAGGCACC AGGGAAGGGG CTGGAGTGGC 30

(2) INFORMATION FOR SEQ ID NO: 33:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..25
(D) OTHER INFORMATION:/function= "backward PCR primer
(25mer), heavy chain"
/product= "Reshaped MAb15, variable region,
version B"

(xi) SEQUENCE DESCRIPTION: SEQ ID NO: 33:

CCCTGGTGCC TGGCAGACCC AAGAC 25

(2) INFORMATION FOR SEQ ID NO: 34:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..20
(D) OTHER INFORMATION:/function= "forward PCR primer
(20mer), heavy chain"
/product= "Reshaped MAb 15, variable region,
version C"

(xi) SEQUENCE DESCRIPTION: SEQ ID NO: 34:

CTCCGGATTC GCCTTTAGCG 20

(2) INFORMATION FOR SEQ ID NO: 35:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(ix) FEATURE:
(A) NAME/KEY: misc_feature
(B) LOCATION:1..20
(D) OTHER INFORMATION:/function= "backward PCR primer
(20mer), heavy chain"
/product= "Reshaped MAb 15, variable region,
version C"

(xi) SEQUENCE DESCRIPTION: SEQ ID NO: 35:

CGCTAAAGGC GAATCCGGAG 20

(2) INFORMATION FOR SEQ ID NO: 36:

(i) SEQUENCE CHARACTERISTICS:
(A) LENGTH: 460 base pairs
(B) TYPE: nucleic acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: sig_peptide
(B) LOCATION:1..72

(ix) FEATURE:
(A) NAME/KEY: CDS
(B) LOCATION:73..411
(D) OTHER INFORMATION:/function= "FR's, CDR's"
/product= "Mouse MAb 15 light chain variable
region"

(xi) SEQUENCE DESCRIPTION: SEQ ID NO: 36:

ATGGGCATCA AGATGGAGTC ACATTCCCTG GTTCTTATGT TACTGCTGCT ATGGGTATCT 60

GGTACCTGTG GG GAC ATT GTG ATG TCA CAG TCT CCA TCC TCC CTA GCT 108
Asp Ile Val Met Ser Gln Ser Pro Ser Ser Leu Ala
20 25

GTG TCA GTT GGA GAG AAG GTT ACT ATG AGC TGC AAG TCC AGT CAG AGC 156
Val Ser Val Gly Glu Lys Val Thr Met Ser Cys Lys Ser Ser Gln Ser
30 35 40 45

CTT TTA TAT AGT AGC AAT CAA AAG AAC TAC TTG GCC TGG TAC CAG CAG 204
Leu Leu Tyr Ser Ser Asn Gln Lys Asn Tyr Leu Ala Trp Tyr Gln Gln
50 55 60

AAA CCA GGG CAG TCT CCT AAA CTG CTG ATT TAC TGG GCA TCC ACT AGG 252
Lys Pro Gly Gln Ser Pro Lys Leu Leu Ile Tyr Trp Ala Ser Thr Arg
65 70 75

GAA TCT GGG GTC CCT GAT CGC TTC ACA GGC AGT GGA TCT GGG ACA GAT 300
Glu Ser Gly Val Pro Asp Arg Phe Thr Gly Ser Gly Ser Gly Thr Asp

80                    85                    90

TTC ACT CTC ACC ATC AGC AGC GTG AAG GCT GAA GAC CCG GCA GTT TAT          348
Phe Thr Leu Thr Ile Ser Ser Val Lys Ala Glu Asp Pro Ala Val Tyr
     95                  100                 105

TAC TGT CAG CAA TAT TCT AAC TAT CCT CCG ACG TTC GGT GGA GGC ACC          396
Tyr Cys Gln Gln Tyr Ser Asn Tyr Pro Pro Thr Phe Gly Gly Gly Thr
110                 115                 120                 125

AAG CTG GAA ATC AAA CGGGCTGATG CTGCACCAAC TGTATCCATC TTCCCACCAT          451
Lys Leu Glu Ile Lys
                130

CCACCCGGG                                                                460

(2) INFORMATION FOR SEQ ID NO: 37:

(i) SEQUENCE CHARACTERISTICS:
(A) LENGTH: 113 amino acids
(B) TYPE: amino acid
(D) TOPOLOGY: linear

(ii) MOLECULE TYPE: protein
(xi) SEQUENCE DESCRIPTION: SEQ ID NO: 37:

Asp Ile Val Met Ser Gln Ser Pro Ser Ser Leu Ala Val Ser Val Gly
  1               5                  10                  15

Glu Lys Val Thr Met Ser Cys Lys Ser Ser Gln Ser Leu Leu Tyr Ser
             20                  25                  30

Ser Asn Gln Lys Asn Tyr Leu Ala Trp Tyr Gln Gln Lys Pro Gly Gln
         35                  40                  45

Ser Pro Lys Leu Leu Ile Tyr Trp Ala Ser Thr Arg Glu Ser Gly Val
     50                  55                  60

Pro Asp Arg Phe Thr Gly Ser Gly Ser Gly Thr Asp Phe Thr Leu Thr
 65                  70                  75                  80

Ile Ser Ser Val Lys Ala Glu Asp Pro Ala Val Tyr Tyr Cys Gln Gln
                 85                  90                  95

Tyr Ser Asn Tyr Pro Pro Thr Phe Gly Gly Gly Thr Lys Leu Glu Ile
            100                 105                 110

Lys

(2) INFORMATION FOR SEQ ID NO: 38:

(i) SEQUENCE CHARACTERISTICS:
(A) LENGTH: 457 base pairs
(B) TYPE: nucleic acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: sig_peptide
(B) LOCATION:1..57

(ix) FEATURE:
(A) NAME/KEY: CDS
(B) LOCATION:58..420
(D) OTHER INFORMATION:/product= "Mous MAb 15, heavy chain
variable region"

(xi) SEQUENCE DESCRIPTION: SEQ ID NO: 38:

ATGACCTTTG GGCTCAGCTT GATTTTCCTT GTCCTTACTT TAAAAGGTGT GAAGTGT 57

GAA GTG CAG GTG GTG GAG TCT GGG GGA GGC TTA GTG AAG CCT GGA GGG 105
Glu Val Gln Val Val Glu Ser Gly Gly Gly Leu Val Lys Pro Gly Gly
115 120 125

TCC CTG AAA CTC TCC TGT GCA GCC TCT GGA TTC GCT TTC AGT GAC TAT 153
Ser Leu Lys Leu Ser Cys Ala Ala Ser Gly Phe Ala Phe Ser Asp Tyr
130 135 140 145

GAC ATG TCT TGG GTT CGC CAG ACT CCG GAG AAG AAG CTG GAG TGG GTC 201
Asp Met Ser Trp Val Arg Gln Thr Pro Glu Lys Lys Leu Glu Trp Val
150 155 160

GCA TAC CTT AGT AGA GGT GGT GGT AGC ACC TAC TAT CCA GAC ACT GTG 249
Ala Tyr Leu Ser Arg Gly Gly Gly Ser Thr Tyr Tyr Pro Asp Thr Val
165 170 175

AAG GGC CGA TTC ACC ATC TCC AGA GAC AAT GCC AAG AAA ATT CTG TTC 297
Lys Gly Arg Phe Thr Ile Ser Arg Asp Asn Ala Lys Lys Ile Leu Phe
180 185 190

CTG CAA ATG ACC AGT CTG AAG TCT GAA GAC GCA GCC ATG TAT TAC TGT 345

Leu Gln Met Thr Ser Leu Lys Ser Glu Asp Ala Ala Met Tyr Tyr Cys
    195                 200                 205

GCA AGA CAT GGT GAG GAG GTC CGC CCC TGG TTT GAT TAC TGG GGC CAA     393
Ala Arg His Gly Glu Glu Val Arg Pro Trp Phe Asp Tyr Trp Gly Gln
210                 215                 220                 225

GGG ACT CTG GTC ACT GTC TCT GCA GCC AAAACGACAC CCCCATCTGT     440
Gly Thr Leu Val Thr Val Ser Ala Ala
                230

CTATCCACTG GCCCGGG     457

(2) INFORMATION FOR SEQ ID NO: 39:

    (i) SEQUENCE CHARACTERISTICS:
        (A) LENGTH: 121 amino acids
        (B) TYPE: amino acid
        (D) TOPOLOGY: linear

    (ii) MOLECULE TYPE: protein
    (xi) SEQUENCE DESCRIPTION: SEQ ID NO: 39:

Glu Val Gln Val Val Glu Ser Gly Gly Gly Leu Val Lys Pro Gly Gly
  1               5                  10                  15

Ser Leu Lys Leu Ser Cys Ala Ala Ser Gly Phe Ala Phe Ser Asp Tyr
             20                  25                  30

Asp Met Ser Trp Val Arg Gln Thr Pro Glu Lys Lys Leu Glu Trp Val
         35                  40                  45

Ala Tyr Leu Ser Arg Gly Gly Gly Ser Thr Tyr Tyr Pro Asp Thr Val
     50                  55                  60

Lys Gly Arg Phe Thr Ile Ser Arg Asp Asn Ala Lys Lys Ile Leu Phe
 65                  70                  75                  80

Leu Gln Met Thr Ser Leu Lys Ser Glu Asp Ala Ala Met Tyr Tyr Cys
                 85                  90                  95

Ala Arg His Gly Glu Glu Val Arg Pro Trp Phe Asp Tyr Trp Gly Gln
            100                 105                 110

Gly Thr Leu Val Thr Val Ser Ala Ala
        115                 120

(2) INFORMATION FOR SEQ ID NO: 40:

    (i) SEQUENCE CHARACTERISTICS:
        (A) LENGTH: 439 base pairs
        (B) TYPE: nucleic acid

(C) STRANDEDNESS: single

(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: misc_signal
(B) LOCATION:1..15

(ix) FEATURE:
(A) NAME/KEY: sig_peptide
(B) LOCATION:16..87

(ix) FEATURE:
(A) NAME/KEY: CDS
(B) LOCATION:88..426
(D) OTHER INFORMATION:/function= "FR's, CDR's"
/product= "Chimeric MAb 15, light chain variable
region"

(xi) SEQUENCE DESCRIPTION: SEQ ID NO: 40:

AAGCTTGCCG CCACCATGGG CATCAAGATG GAGTCACAGA CTCTGGTTCT TATGTTACTG 60

CTGCTATGGG TATCTGGTAC CTGTGGG GAC ATT GTG ATG TCA CAG TCT CCA 111
Asp Ile Val Met Ser Gln Ser Pro
125

TCC TCC CTA GCT GTG TCA GTT GGA GAG AAG GTT ACT ATG AGC TGC AAG 159
Ser Ser Leu Ala Val Ser Val Gly Glu Lys Val Thr Met Ser Cys Lys
130 135 140 145

TCC AGT CAG AGC CTT TTA TAT AGT AGC AAT CAA AAG AAC TAC TTG GCC 207
Ser Ser Gln Ser Leu Leu Tyr Ser Ser Asn Gln Lys Asn Tyr Leu Ala
150 155 160

TGG TAC CAG CAG AAA CCA GGG CAG TCT CCT AAA CTG CTG ATT TAC TGG 255
Trp Tyr Gln Gln Lys Pro Gly Gln Ser Pro Lys Leu Leu Ile Tyr Trp
165 170 175

GCA TCC ACT AGG GAA TCT GGG GTC CCT GAT CGC TTC ACA GGC AGT GGA 303
Ala Ser Thr Arg Glu Ser Gly Val Pro Asp Arg Phe Thr Gly Ser Gly
180 185 190

TCT GGG ACA GAT TTC ACT CTC ACC ATC AGC AGC GTG AAG GCT GAA GAC 351
Ser Gly Thr Asp Phe Thr Leu Thr Ile Ser Ser Val Lys Ala Glu Asp
195 200 205

CCG GCA GTT TAT TAC TGT CAG CAA TAT TCT AAC TAT CCT CCG ACG TTC 399
Pro Ala Val Tyr Tyr Cys Gln Gln Tyr Ser Asn Tyr Pro Pro Thr Phe
210 215 220 225

GGT GGA GGC ACC AAG CTG GAA ATC AAA CGTGAGTGGA TCC 439
Gly Gly Gly Thr Lys Leu Glu Ile Lys
230

(2) INFORMATION FOR SEQ ID NO: 41:

(i) SEQUENCE CHARACTERISTICS:
(A) LENGTH: 113 amino acids
(B) TYPE: amino acid
(D) TOPOLOGY: linear

(ii) MOLECULE TYPE: protein
(xi) SEQUENCE DESCRIPTION: SEQ ID NO: 41:

Asp Ile Val Met Ser Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Pro Ala Val Tyr Tyr Cys Gln Gln
                85                  90                  95

Tyr Ser Asn Tyr Pro Pro Thr Phe Gly Gly Gly Thr Lys Leu Glu Ile
            100                 105                 110

Lys

(2) INFORMATION FOR SEQ ID NO: 42:

(i) SEQUENCE CHARACTERISTICS:
(A) LENGTH: 441 base pairs
(B) TYPE: nucleic acid
(C) STRANDEDNESS: single

(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: misc_signal
(B) LOCATION:1..11

(ix) FEATURE:
(A) NAME/KEY: sig_peptide
(B) LOCATION:12..68

(ix) FEATURE:
(A) NAME/KEY: CDS
(B) LOCATION:69..428
(D) OTHER INFORMATION:/function= "FR's, CDR's"
/product= "Chimeric MAb 15, heavy chain variable
region"

(xi) SEQUENCE DESCRIPTION: SEQ ID NO: 42:

AAGCTTCCAC CATGAACTTT GGGCTCAGCT TGATTTTCCT TGTCCTTACT TTAAAAGGTG 60

TGAAGTGT GAA GTG CAG GTG GTG GAG TCT GGG GGA GGC TTA GTG AAG CCT 110
Glu Val Gln Val Val Glu Ser Gly Gly Gly Leu Val Lys Pro
115 120 125

GGA GGG TCC CTG AAA CTC TCC TGT GCA GCC TCT GGA TTC GCT TTC AGT 158
Gly Gly Ser Leu Lys Leu Ser Cys Ala Ala Ser Gly Phe Ala Phe Ser
130 135 140

GAC TAT GAC ATG TCT TGG GTT CGC CAG ACT CCG GAG AAG AGG CTG GAG 206
Asp Tyr Asp Met Ser Trp Val Arg Gln Thr Pro Glu Lys Arg Leu Glu
145 150 155

TGG GTC GCA TAC CTT AGT AGA GGT GGT GGT AGC ACC TAC TAT CCA GAC 254
Trp Val Ala Tyr Leu Ser Arg Gly Gly Gly Ser Thr Tyr Tyr Pro Asp
160 165 170 175

ACT GTG AAG GGC CGA TTC ACC ATC TCC AGA GAC AAT GCC AAG AAA ATT 302
Thr Val Lys Gly Arg Phe Thr Ile Ser Arg Asp Asn Ala Lys Lys Ile
180 185 190

CTG TTC CTG CAA ATG ACC AGT CTG AAG TCT GAA GAC GCA GCC ATG TAT 350
Leu Phe Leu Gln Met Thr Ser Leu Lys Ser Glu Asp Ala Ala Met Tyr

195 200 205

TAC TGT GCA AGA CAT GGT GAG GAG GTC CGC CCC TGG TTT GAT TAC TGG 398
Tyr Cys Ala Arg His Gly Glu Glu Val Arg Pro Trp Phe Asp Tyr Trp
210 215 220

GGC CAA GGG ACT CTG GTC ACT GTC TCT GCA GGTGAGTGGA TCC 441
Gly Gln Gly Thr Leu Val Thr Val Ser Ala
225 230

(2) INFORMATION FOR SEQ ID NO: 43:

(i) SEQUENCE CHARACTERISTICS:
(A) LENGTH: 120 amino acids
(B) TYPE: amino acid
(D) TOPOLOGY: linear

(ii) MOLECULE TYPE: protein
(xi) SEQUENCE DESCRIPTION: SEQ ID NO: 43:

Glu Val Gln Val Val Glu Ser Gly Gly Gly Leu Val Lys Pro Gly Gly
1               5                   10                  15

Ser Leu Lys Leu Ser Cys Ala Ala Ser Gly Phe Ala Phe Ser Asp Tyr
            20                  25                  30

Asp Met Ser Trp Val Arg Gln Thr Pro Glu Lys Arg Leu Glu Trp Val
        35                  40                  45

Ala Tyr Leu Ser Arg Gly Gly Gly Ser Thr Tyr Tyr Pro Asp Thr Val
    50                  55                  60

Lys Gly Arg Phe Thr Ile Ser Arg Asp Asn Ala Lys Lys Ile Leu Phe
65                  70                  75                  80

Leu Gln Met Thr Ser Leu Lys Ser Glu Asp Ala Ala Met Tyr Tyr Cys
                85                  90                  95

Ala Arg His Gly Glu Glu Val Arg Pro Trp Phe Asp Tyr Trp Gly Gln
            100                 105                 110

Gly Thr Leu Val Thr Val Ser Ala
        115                 120

(2) INFORMATION FOR SEQ ID NO: 44:

(i) SEQUENCE CHARACTERISTICS:
(A) LENGTH: 113 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: Protein
(B) LOCATION:1..113
(D) OTHER INFORMATION:/note= "MAb15 light chain variable
region, Vl15 version (Fig. 7)"

(xi) SEQUENCE DESCRIPTION: SEQ ID NO: 44:

Asp Ile Val Met Ser Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Pro Ala Val Tyr Tyr Cys Gln Gln
                85                  90                  95

Tyr Ser Asn Tyr Pro Pro Thr Phe Gly Gly Gly Thr Lys Leu Glu Ile
            100                 105                 110

Lys

(2) INFORMATION FOR SEQ ID NO: 45:

(i) SEQUENCE CHARACTERISTICS:
(A) LENGTH: 113 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: Peptide
(B) LOCATION:1..113
(D) OTHER INFORMATION:/note= "Reshaped MAb 15 light
chain, variable region RV1a version Fig.7"

(xi) SEQUENCE DESCRIPTION: SEQ ID NO: 45:

Asp Ile Gln Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Tyr Trp Ala Ser Thr Arg Glu Ser Gly Val Pro
    50                  55                  60

Asp Arg Phe Ser Gly Ser Gly Ser Gly Thr Asp Phe Thr Phe Thr Ile
65                  70                  75                  80

Ser Ser Leu Gln Pro Glu Asp Ile Ala Thr Val Tyr Tyr Cys Gln Gln
                85                  90                  95

Tyr Ser Asn Tyr Pro Pro Thr Phe Gly Gln Gly Thr Lys Val Glu Ile
            100                 105                 110

Lys

(2) INFORMATION FOR SEQ ID NO: 46:

(i) SEQUENCE CHARACTERISTICS:
(A) LENGTH: 80 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: Peptide
(B) LOCATION:1..80
(D) OTHER INFORMATION:/note= "Framework region of human
REI"

(xi) SEQUENCE DESCRIPTION: SEQ ID NO: 46:

Asp Ile Gln Met Thr Gln Ser Pro Ser Ser Leu Ser Ala Ser Val Gly
1               5                   10                  15

Asp Arg Val Thr Ile Thr Cys Trp Tyr Gln Gln Lys Pro Gly Lys Ala
            20                  25                  30

Pro Lys Leu Leu Ile Tyr Gly Val Pro Ser Arg Phe Ser Gly Ser Gly
        35                  40                  45

Ser Gly Thr Asp Phe Thr Phe Thr Ile Ser Ser Leu Gln Pro Glu Asp
    50                  55                  60

Ile Ala Thr Tyr Tyr Cys Phe Gly Gln Gly Thr Lys Val Glu Ile Lys
65                  70                  75                  80

(2) INFORMATION FOR SEQ ID NO: 47:

(i) SEQUENCE CHARACTERISTICS:
(A) LENGTH: 120 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: Peptide
(B) LOCATION:1..120
(D) OTHER INFORMATION:/note= "MAb 15 heavy chain variable
region, VH15 version"

(xi) SEQUENCE DESCRIPTION: SEQ ID NO: 47:

Glu Val Gln Val Val Glu Ser Gly Gly Gly Leu Val Lys Pro Gly Gly
1               5                   10                  15

Ser Leu Lys Leu Ser Cys Ala Ala Ser Gly Phe Ala Phe Ser Asp Tyr
            20                  25                  30

Asp Met Ser Trp Val Arg Gln Thr Pro Glu Lys Arg Leu Glu Trp Val
        35                  40                  45

Ala Tyr Leu Ser Arg Gly Gly Gly Ser Thr Tyr Tyr Pro Asp Thr Val
    50                  55                  60

Lys Gly Arg Phe Thr Ile Ser Arg Asp Asn Ala Lys Lys Ile Leu Phe
65                  70                  75                  80

Leu Gln Met Thr Ser Leu Lys Ser Glu Asp Ala Ala Met Tyr Tyr Cys
                85                  90                  95

Ala Arg His Gly Glu Glu Val Arg Pro Trp Phe Asp Tyr Trp Gly Gln
            100                 105                 110

Gly Thr Leu Val Thr Val Ser Ala
        115                 120

(2) INFORMATION FOR SEQ ID NO: 48:

(i) SEQUENCE CHARACTERISTICS:
(A) LENGTH: 120 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: Peptide
(B) LOCATION:1..120
(D) OTHER INFORMATION:/note= "Reshaped MAb 15, heavy
chain variabel region, RVHa version"

(xi) SEQUENCE DESCRIPTION: SEQ ID NO: 48:

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Asp Met Ser Trp Val Arg Gln Ala Pro Gly Lys Arg Leu Glu Trp Val
        35                  40                  45

Ala Tyr Leu Ser Arg Gly Gly Gly Ser Thr Tyr Tyr Pro Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Gly Glu Glu Val Arg Pro Trp Phe Asp Tyr Trp Gly Gln
            100                 105                 110

Gly Thr Leu Val Thr Val Ser Ser
        115                 120

INFORMATION FOR SEQ ID NO: 49:

(i) SEQUENCE CHARACTERISTICS:
(A) LENGTH: 120 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: Peptide
(B) LOCATION:1..120
(D) OTHER INFORMATION:/note= "Reshaped MAb 15, heavy
chain variable region, RVHb version"

(xi) SEQUENCE DESCRIPTION: SEQ ID NO: 49:

Glu Val Gln Leu Leu Glu Ser Gly Gly Gly Leu Val Gln Pro Gly Gly

1                   5                   10                  15

Ser Leu Arg Leu Ser Cys Ala Ala Ser Gly Phe Thr Phe Ser Asp Tyr
            20                  25                  30

Asp Met Ser Trp Val Arg Gln Ala Pro Gly Lys Gly Leu Glu Trp Val
        35                  40                  45

Ala Tyr Leu Ser Arg Gly Gly Gly Ser Thr Tyr Tyr Pro Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Gly Glu Glu Val Arg Pro Trp Phe Asp Tyr Trp Gly Gln
            100                 105                 110

Gly Thr Leu Val Thr Val Ser Ser
        115                 120

(2) INFORMATION FOR SEQ ID NO: 50:

(i) SEQUENCE CHARACTERISTICS:
(A) LENGTH: 120 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: Peptide
(B) LOCATION:1..120
(D) OTHER INFORMATION:/note= "Reshaped MAb 15, heavy
chain variable region, RVHc version"

(xi) SEQUENCE DESCRIPTION: SEQ ID NO: 50:

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Ala Phe Ser Asp Tyr

20                  25                  30

Asp Met Ser Trp Val Arg Gln Ala Pro Gly Lys Gly Leu Glu Trp Val
        35                  40                  45

Ala Tyr Leu Ser Arg Gly Gly Gly Ser Thr Tyr Tyr Pro Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Gly Glu Glu Val Arg Pro Trp Phe Asp Tyr Trp Gly Gln
            100                 105                 110

Gly Thr Leu Val Thr Val Ser Ser
        115                 120

INFORMATION FOR SEQ ID NO: 51:

(i) SEQUENCE CHARACTERISTICS:
(A) LENGTH: 87 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: Peptide
(B) LOCATION:1..87
(D) OTHER INFORMATION:/note= "Framework region of human
antibody 30PI"

(xi) SEQUENCE DESCRIPTION: SEQ ID NO: 51:

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Trp Val
            20                  25                  30

Arg Gln Ala Pro Gly Lys Gly Leu Glu Trp Val Ser Arg Phe Thr Ile

35                    40                    45

Ser Arg Asp Asn Ser Lys Asn Thr Leu Tyr Leu Gln Met Asn Ser Leu
    50                  55                  60

Arg Ala Glu Asp Thr Ala Val Tyr Tyr Cys Ala Lys Trp Gly Gln Gly
65                  70                  75                  80

Thr Leu Val Thr Val Ser Ser
                85

(2) INFORMATION FOR SEQ ID NO: 52:

(i) SEQUENCE CHARACTERISTICS:
(A) LENGTH: 530 base pairs
(B) TYPE: nucleic acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: misc_signal
(B) LOCATION:1..13

(ix) FEATURE:
(A) NAME/KEY: sig_peptide
(B) LOCATION:14..152

(ix) FEATURE:
(A) NAME/KEY: CDS
(B) LOCATION:153..491
(D) OTHER INFORMATION:/function= "FR's and CDR's"
/product= "Reshaped MAB 15, light chain variable
region"

(xi) SEQUENCE DESCRIPTION: SEQ ID NO: 52:

AAGCTTCGCC ACCATGGGAT GGAGCTGTAT CATCCTCTTC TTGGTAGCAA CAGCTACAGG     60

TAAGGGGCTC ACAGTAGCAG GCTTGAGGTC TGGACATATA TATGGGTGAC AATGACATCC    120

ACTTTGCCTT TCTCTCCACA GGTGTCCACT CC GAC ATC CAG ATG ACC CAG AGC      173
                                    Asp Ile Gln Met Thr Gln Ser
                                                        125

CCA AGC AGC CTG AGC GCC AGC GTG GGT GAC AGA GTG ACC ATC ACC TGT 221
Pro Ser Ser Leu Ser Ala Ser Val Gly Asp Arg Val Thr Ile Thr Cys
130 135 140

AAG TCC AGT CAG AGC CTT TTA TAT AGT AGC AAT CAA AAG AAC TAC TTG 269
Lys Ser Ser Gln Ser Leu Leu Tyr Ser Ser Asn Gln Lys Asn Tyr Leu
145 150 155

GCC TGG TAC CAG CAG AAG CCA GGT AAG GCT CCA AAG CTG CTG ATC TAC 317
Ala Trp Tyr Gln Gln Lys Pro Gly Lys Ala Pro Lys Leu Leu Ile Tyr
160 165 170 175

TGG GCA TCC ACT AGG GAA TCT GGT GTG CCA GAC AGA TTC AGC GGT AGC 365
Trp Ala Ser Thr Arg Glu Ser Gly Val Pro Asp Arg Phe Ser Gly Ser
180 185 190

GGT AGC GGT ACC GAC TTC ACC TTC ACC ATC AGC AGC CTC CAG CCA GAG 413
Gly Ser Gly Thr Asp Phe Thr Phe Thr Ile Ser Ser Leu Gln Pro Glu
195 200 205

GAC ATC GCT ACC TAC TAC TGC CAG CAA TAT TCT AAC TAT CCT CCG ACG 461
Asp Ile Ala Thr Tyr Tyr Cys Gln Gln Tyr Ser Asn Tyr Pro Pro Thr
210 215 220

TTC GGC CAA GGG ACC AAA GTG GAA ATC AAA CGTGAGTAGA ATTTAAACTT 511
Phe Gly Gln Gly Thr Lys Val Glu Ile Lys
225 230

TGCTTCCTCA GTTGGATCC 530

(2) INFORMATION FOR SEQ ID NO: 53:

(i) SEQUENCE CHARACTERISTICS:
(A) LENGTH: 113 amino acids
(B) TYPE: amino acid
(D) TOPOLOGY: linear

(ii) MOLECULE TYPE: protein
(xi) SEQUENCE DESCRIPTION: SEQ ID NO: 53:

Asp Ile Gln Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Tyr Ser
            20                  25                  30

Ser Asn Gln Lys Asn Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val

    50                  55                  60

Pro Asp Arg Phe Ser Gly Ser Gly Ser Gly Thr Asp Phe Thr Phe Thr
65                  70                  75                  80

Ile Ser Ser Leu Gln Pro Glu Asp Ile Ala Thr Tyr Tyr Cys Gln Gln
                85                  90                  95

Tyr Ser Asn Tyr Pro Pro Thr Phe Gly Gln Gly Thr Lys Val Glu Ile
            100                 105                 110

Lys

(2) INFORMATION FOR SEQ ID NO: 54:

(i) SEQUENCE CHARACTERISTICS:
(A) LENGTH: 445 base pairs
(B) TYPE: nucleic acid
(C) STRANDEDNESS: single
(D) TOPOLOGY: linear

(ii) MOLECULE TYPE: peptide

(iii) HYPOTHETICAL: NO

(iv) ANTI-SENSE: NO

(v) FRAGMENT TYPE: N-terminal

(ix) FEATURE:
(A) NAME/KEY: misc_signal
(B) LOCATION:1..15

(ix) FEATURE:
(A) NAME/KEY: sig_peptide
(B) LOCATION:16..72

(ix) FEATURE:
(A) NAME/KEY: CDS
(B) LOCATION:73..432
(D) OTHER INFORMATION:/function= "FR's, CDR's"
/product= "Reshaped MAb 15, heavy chain variable
region"

(xi) SEQUENCE DESCRIPTION: SEQ ID NO: 54:

AAGCTTGCCG CCACCATGGA GTTTGGGCTG AGCTGGCTTT TTCTTGTGGC TATTTTAAAA 60

GGTGTCCAGT GT GAG GTG CAG CTG TTG GAG TCT GGG GGA GGC TTG GTA 108
Glu Val Gln Leu Leu Glu Ser Gly Gly Gly Leu Val

115 120 125

CAG CCT GGG GGG TCC CTG AGA CTC TCC TGT GCA GCC TCC GGA TTC ACC 156
Gln Pro Gly Gly Ser Leu Arg Leu Ser Cys Ala Ala Ser Gly Phe Thr
130 135 140

TTT AGC GAC TAT GAC ATG TCT TGG GTC CGC CAG GCA CCA GGG AAG AGG 204
Phe Ser Asp Tyr Asp Met Ser Trp Val Arg Gln Ala Pro Gly Lys Arg
145 150 155

CTG GAG TGG GTC GCA TAC CTT AGT AGA GGT GGT GGT AGC ACC TAC TAT 252
Leu Glu Trp Val Ala Tyr Leu Ser Arg Gly Gly Gly Ser Thr Tyr Tyr
160 165 170

CCA GAC ACT GTG AAG GGC CGA TTC ACC ATC TCG AGA GAC AAT TCC AAG 300
Pro Asp Thr Val Lys Gly Arg Phe Thr Ile Ser Arg Asp Asn Ser Lys
175 180 185

AAC ACG CTG TAT CTG CAA ATG AAC AGC CTG AGA GCC GAG GAC ACG GCC 348
Asn Thr Leu Tyr Leu Gln Met Asn Ser Leu Arg Ala Glu Asp Thr Ala
190 195 200 205

GTA TAT TAC TGT GCG AGA CAT GGT GAG GAG GTC CGC CCC TGG TTT GAT 396
Val Tyr Tyr Cys Ala Arg His Gly Glu Glu Val Arg Pro Trp Phe Asp
210 215 220

TAC TGG GGC CAG GGA ACC CTG GTC ACC GTC TCC TCA GGTGAGTGGA 442
Tyr Trp Gly Gln Gly Thr Leu Val Thr Val Ser Ser
225 230

TCC 445

(2) INFORMATION FOR SEQ ID NO: 55:

(i) SEQUENCE CHARACTERISTICS:
(A) LENGTH: 120 amino acids
(B) TYPE: amino acid
(D) TOPOLOGY: linear

(ii) MOLECULE TYPE: protein
(xi) SEQUENCE DESCRIPTION: SEQ ID NO: 55:

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Asp Met Ser Trp Val Arg Gln Ala Pro Gly Lys Arg Leu Glu Trp Val
        35                  40                  45

Ala Tyr Leu Ser Arg Gly Gly Gly Ser Thr Tyr Tyr Pro Asp Thr Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Gly Glu Glu Val Arg Pro Trp Phe Asp Tyr Trp Gly Gln
            100                 105                 110

Gly Thr Leu Val Thr Val Ser Ser
        115                 120

## Description

The invention relates to a new monoclonal antibody which was humanized from its murine form. The invention relates, especially, to the humanized and reshaped varient of murine MAb 15 (DSM ACC2117) which is known to show a therapeutical effect on human tumor cells, preferrably on human lung cancer.

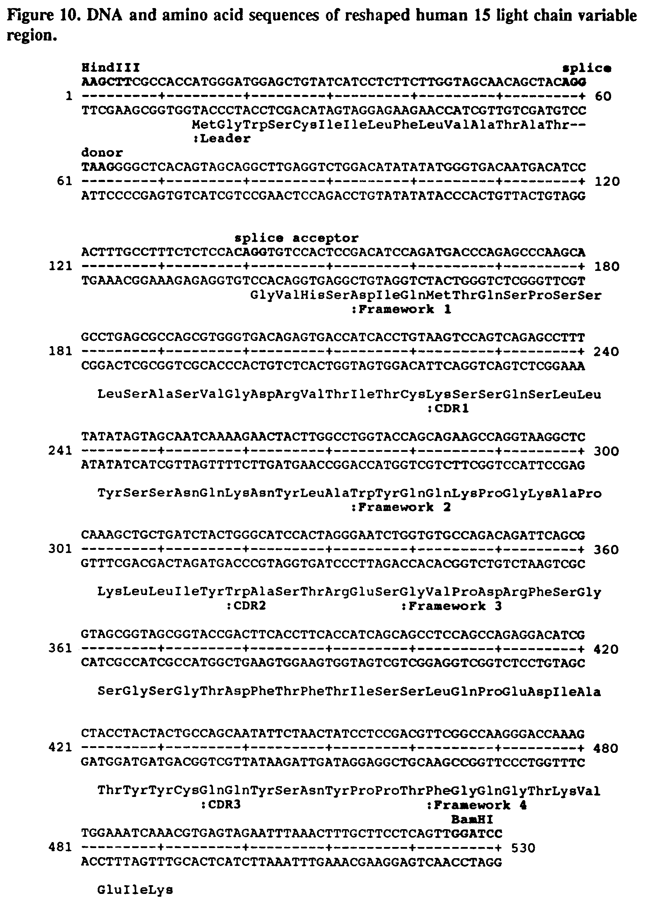

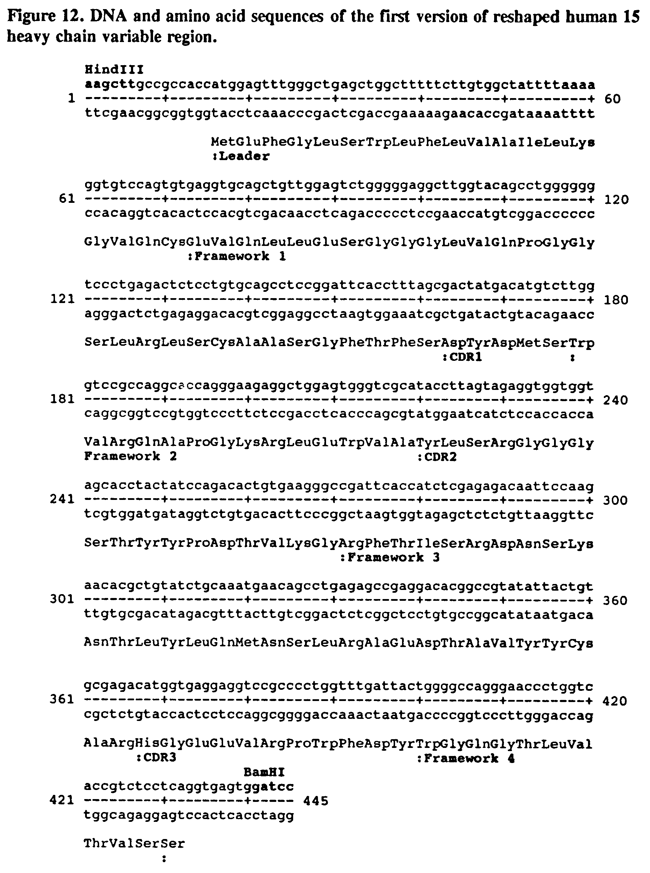

Furthermore, the invention relates to specific DNA- and amino acid sequences of the variable regions of said antibody (Figures 10 and 12). The invention relates, moreover, to pharmaceutical compositions comprising said antibodies or fragments thereof for the purposes of treating tumors like melanoma, glioma or carcinoma, especially lung carcinoma. The said antibodies or fragments can be used also for diagnostic applications regarding locating and assessing the said tumors in vitro or in vivo.

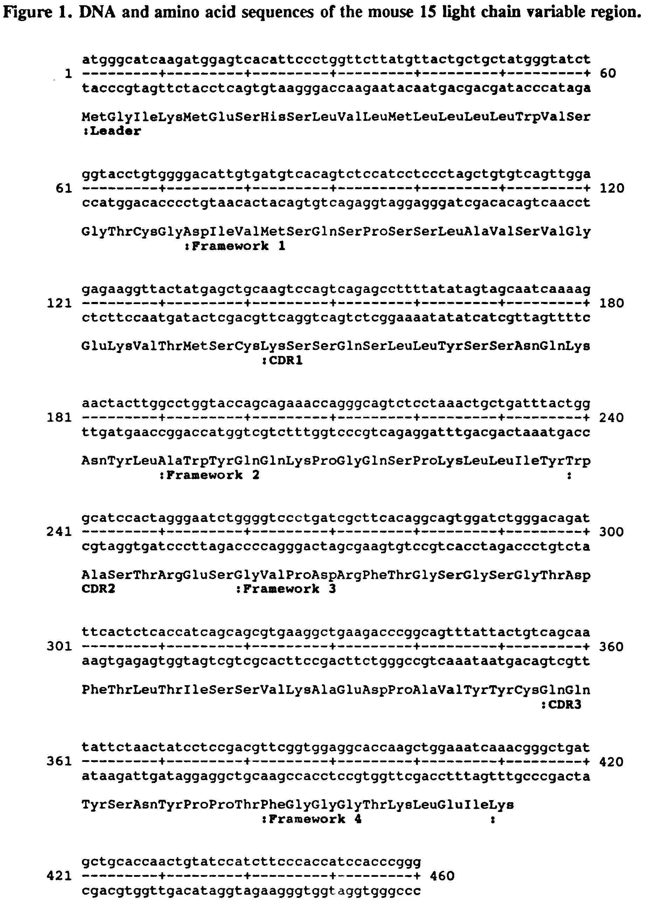

Finally, the invention relates to the DNA- and amino acid sequence of the murin Mab 15 (Fig. 1), which was not known up to date.

### Technical Field of the Invention

Mouse monoclonal antibody MAb 15 was isolated from mice immunized with TKB-2 cells, a variant cell line of human small cell lung carcinoma.

MAb 15 was deposited under the assession number DSM ACC2117 by applicant at "Deutsche Sammlung für Mikroorganismen" according to the rules and requirements of Budapest Treaty.

MAb 15 was originally reported to react with four major types of lung cancer cells and 72 % of lung cancers. Cross-reactions with normal tissues were limited to esophagus and renules tubules (Endo et al., 1986). MAb 15 was reported to be a mouse IgG1/kappa antibody that recognized two glycoproteins of molecular weights 45,000 and 85,000 kD (Endo et al., 1986). The association constant for the whole mouse IgG antibody was determined to be 1.9 X 10⁻⁹ M (Endo et al., 1988). Experiments with mice with xenografts of TKB-2 cells showed that MAb 15 and MAb 15-derived antibody fragments showed antibody localization to the tumor cells (Endo et al., 1988). Further work demonstrated that MAb 15, when added to culture medium, inhibited the proliferation of lung cancer cells. It was proposed that MAb 15 inhibited growth by blocking a growth factor receptor specific to lung cancer (Kamma et al., 1989).

The antigen recognized by MAb 15 is now thought to be the human cell surface antigen 4F2, a 120-125 kD disulfide-linked heterodimer consisting of a 80-90 kDa glycosylated heavy chain and a 38 kD nonglycosylated light chain. The antigen is ubiquitously expressed on proliferating cells but only in resting cells from certain tissues. Its function has been proposed to relate to Ca⁺/Na⁺ exchange. It is thought that 4F2 has a role in the regulation of intracellular calcium concentration and the concomitant control of growth, excitability, and endocrine secretion (Teixeira et al., 1987; Quackenbush et al., 1987).

MAb 15 is one of the mouse antibodies which couls be a possible basis for the immunotherapy of solid tumors. Mouse antibodies, however, are highly immunogenic in humans and, for this reason, their therapeutic value in humans is limited. The half-life of mouse antibodies in vivo in humans is relatively short. In addition, mouse antibodies can not be administered in multiple doses without generating an immune response which not only interferes with the planned efficacy but also risks an adverse allergic response in the patient.

Therefore, it seems to be desirable to develop a humanized version of MAb 15, in order to overcome said disadvantages. Although several humanization procedures are known in the art (e.g. EP 0239 400), the results of these humanization procedures are often not very satisfying because the newly synthesized "human" antibodies show in many cases a partial or complete loss of affinity to the binding site of the antigen. Therefore, it is necessarry to exchange some amino acids above all in the framework regions of the antibody to reestablish antigen affinity. This necessary exchange is, however, normally not predictable.

Mouse antibodies can be humanized in two ways. The more simple method is to construct chimeric antibodies where the variable regions are derived from the original mouse monoclonal antibody and the constant regions are derived from suitable human antibodies. The resulting chimeric antibody contains the entire variable domains of the original mouse antibody and is expected to bind antigen with the same specificity as the original mouse antibody. In addition, chimeric antibodies have a substantial reduction in the percent of the protein sequence derived from a non-human source and, therefor, are expected to be less immunogenic than the original mouse antibody. Although chimeric antibodies are predicted to bind antigen well and to be less immunogenic, an immune response to the mouse variable regions can still occur (LoBuglio et al., 1989: Khazaeli et al., 1991; Meredith et al., 1991; Saleh et al., 1992).

The second method for humanizing mouse antibodies is more complicated but more extensively reduces to potential immunogenicity of the mouse antibody. In this method, the complementarity determining regions (CDRs) from the variable regions of the mouse antibody are grafted into human variable regions to create "reshaped" human variable regions. These reshaped human variable regions are then joined to human constant regions. The only portions of the final reshaped human antibody derived from non-human protein sequences are the CDRs. CDRs are highly variable protein sequences. They do not show species-specific sequences. For these reasons, a reshaped human antibody carrying mouse CDRs should not be any more immunogenic than a natural human antibody containing human CDRs.

This invention comprises (1) to clone and sequence the mouse 15 light and heavy chain variable regions, (2) to construct, express, and analyze chimeric 15 antibody, (3) to model the structure of the mouse 15 variable regions, (4) to design reshaped human 15 variable regions, and (5) to construct, express, and analyze several versions of reshaped human 15 antibodies. A previous report (WO 92/15683) describes in detail the results of the first two sections with respect to the humanization of MAb 425, an antibody which is directed against EGF receptor.

The specification relates to several technical terms which are herewith defined as follows:
"FRs" (framework regions) mean the four subregions of the light or heavy chain variable regions that support the three CDRs.
"CDRs" (complementarity determining regions) mean the three subregions of the light or heavy chain variable regions which have hypervariable sequences and form loop structures that are primarily responsible for making direct contact with antigen.
"Chimeric" or partially humanized antibodies mean antibodies comprising constant regions deriving from human sources and variable regions (CDRs included) deriving from non-human sources, e.g. from the mouse.
"Humanized" or fully humanized antibodies mean antibodies comprising constant regions and FRs deriving from human sources whereas the CDRs derive from non-human sources.
"Reshaped" antibodies mean antibodies comprising FRs in which a few amino acids were changed in order to obtain an optimal conformation and, therefore, to reestablish affinity to the binding site of the antigen.

The invention does not only comprise the expressively mentioned DNA or amino acid sequences but includes, in addition, mutations and variants of these sequences (alterations of a few nucleotides or amino acids), caused, for example, by physical or chemical spontaneous influences or desired treatments, which do not change the biological activity of them or of the complete antibody or antibody fragments.

The term "antibody" comprises also antibody fragments, such as F(ab'), F(ab')₂ or single chain Fv.

### LIST OF TABLES

- Table 1.: PCR primers used for cloning the mouse 15 variable regions.
- Table 2.: Comparison of the mouse 15 variable regions to the consensus sequences for mouse variable regions.
- Table 3.: PCR primers for the construction of chimeric 15 antibody.
- Table 4.: Canonical structure residues for light chain variable region.
- Table 5.: Canonical structure residues for heavy chain variable region.
- Table 6.: Comparisons of mouse 15 light chain variable region to mouse and human light chain variable regions.
- Table 7.: Comparisons of mouse 15 heavy chain variable region to mouse and human heavy chain variable regions.
- Table 8.: Alignment of amino acid sequences leading to the design of reshaped human 15 V_{L} regions.
- Table 9.: Alignment of amino acid sequences leading to the design of reshaped human 15 V_{H} regions.
- Table 10.: PCR primers for the construction of reshaped human 15 light chain variable region.
- Table 11.: PCR primers used for the construction of version A of the reshaped human 15 heavy chain variable region.
- Table 12.: PCR primers used for the construction of versions B and C of the reshaped human 15 heavy chain variable region.

### LIST OF FIGURES

- Figure 1.: DNA and amino acid sequences of the mouse 15 light chain variable region.
- Figure 2.: DNA and amino acid sequences of the mouse 15 heavy chain variable region.
- Figure 3.: Vectors that employ the human cytomegalovirus (HCMV) immediate early gene promoter and enhancer to express genetically engineered antibodies in mammalian cells.
- Figure 4.: DNA and amino acid sequence of the mouse 15 light chain variable region as PCR-modified for use in the construction of chimeric light chain.
- Figure 5.: DNA and amino acid sequences of the mouse 15 heavy chain variable region as PCR-modified for use in the construction of chimeric 15 heavy chain.
- Figure 6.: Molecular model of the variable regions from mouse 15 antibody.
- Figure 7.: Summary of the design of the one version of reshaped human 15 light chain variable region.
- Figure 8.: Summary of the design of the three different versions of reshaped human 15 heavy chain variable region.
- Figure 9.: Construction of reshaped human 15 light chain variable region.
- Figure 10.: DNA and amino acid sequences of reshaped human 15 light chain variable region.
- Figure 11.: Construction of the first version of reshaped human 15 heavy chain variable region.
- Figure 12.: DNA and amino acid sequences of the first version of reshaped human 15 heavy chain variable region.
- Figure 13.: Construction of the second and third versions of reshaped human 15 heavy chain variable regions.
- Figure 14.: ELISA assay testing the ability of chimeric and reshaped human 15 antibodies to bind to A431 cells (unpurified antibody from cos cell supernatants).
- Figure 15.: ELISA assay testing the ability of chimeric and reshaped human 15 antibodies to bind to A431 cells (Protein A-purified antibody from cos cell supernatants).
- Figure 16.: Curve-fitting plot and Kd calculations for mouse 15 antibody binding to A431 cells.
- Figure 17.: Curve-fitting plot and Kd calculations for chimeric 15 antibody binding to A431 cells.
- Figure 18.: Curve-fitting plot and Kd calculations for reshaped human 15 antibody (version L+Ha) binding to A431 cells.

### Detailed Description

**Cloning of the full-length mouse 15 variable regions.** The cloning of mouse 15 variable regions from cDNA prepared from the hybridoma cell line expressing MAb 15 was achieve using mixed degenerate primers designed (Jones and Bendig, 1991). Two sets of primers have been designed. One set hybridizes to the leader sequences of mouse kappa chains and consists of 11 primer groups. The other set hybridizes to the leader sequences of mouse kappa chains and consists of 11 primer groups. The other set hybridizes to the leader sequences of mouse gamma chains and consists of 12 primer groups. The degenerate 5'-PCR primers were used in conjunction with 3'-PCR primers that hybridize within the mouse kappa and gamma-1 constant regions (Table 1). Both sets of primers are external to the variable region coding sequence and thus clone full-length mouse variable regions. After PCR amplification, products of the appropriate length (450 bp) from the kappa and gamma chain reactions were obtained. These products were subsequently cloned into pUC19 vectors using restriction enzyme sites contained within the PCR primers, XmaI within the leader sequence primers and SalI within the constant region primers. Due to the possibility of PCR errors being introduced during the amplification, at least two clones from separate PCR reactions were cloned and sequenced.

The mouse 15 gamma chain variable region was PCR-cloned using a mixture of the gamma leader sequence primers and the gamma-1 constant region primer. DNA sequencing of two heavy chain clones gave identical sequences that could be translated into a full-length variable region. A third cloned PCR fragment gave a sequence with a base substitution at position 376 which resulted in a stop codon within the variable region. This base substition was probably introduced during the PCR amplification. From the DNA sequences obtained, it appeared that mouse heavy chain leader sequence primer group 4 was responsible for PCR-cloning mouse 15 heavy chain variable region (Table 1).

The mouse 15 kappa chain variable region was PCR-cloned using a mixture of the kappa leader sequence primers and the kappa constant region primer. Primer group 2 was responsible for the PCR-cloning of a 450 bp DNA fragment. DNA sequence analysis of four DNA fragments from two separate PCR reaction showed that these kappa chain variable regions had a stop in framework 4 and a reading frame shift around CDR3. The DNA sequence of this non-functional kappa chain corresponded to that of the mopc21n gene sequence (Walfield et al., 1981). This gene has a misalignment which results i a deletion of four nucleotides at the V-J₂ junction and thus results in a translational reading frame shift. PCR-cloning was repeated using the kappa chain leader sequence primer groups separately. DNA products were obtained using primers from group 2 and group 7. The non-functional mopc21n kappa chain was cloned using primers form group 2. A full-length variable region was cloned using primers from group 7. This PCR product was thought to be the functional mouse 15 kappa chain variable region. Identical DNA sequences were obtained from two separate PCR reactions generated using the group 7 primers. They both coded for an identical full-length variable region.

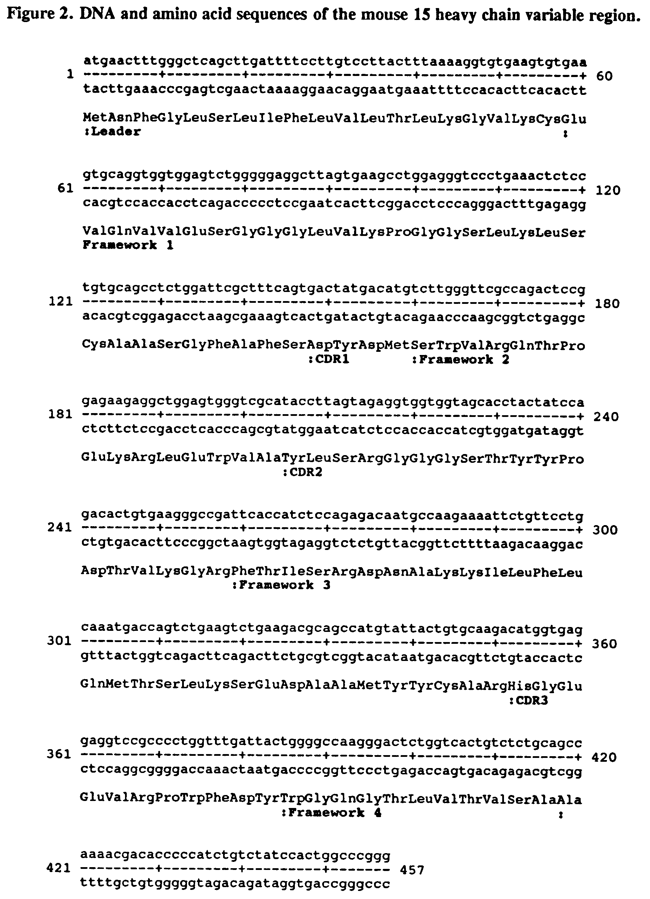

The DNA sequences and protein translations of the mouse 15 light and heavy chain variable regions are shown in Figures 1 and 2 respectively.

**Comparing the protein sequences of mouse 15 variable regions to the consensus sequences of mouse variable regions.** The protein sequences of the mouse 15 light and heavy chain variable regions were compared to the consensus sequences of the different subgroups of mouse variable regions, ad defined by Kapat et al. (1987), using the GAP program from the Sequence Analysis Software Package of the Genetics Computer Group, University of Wisconsin, U.S.A. Percentage identities are shown in Table 2. The mouse 15 light chain variable region is most similar to the mouse kappa chain variable region subgroup "k1" with a 72.6 % identity. The mouse 15 heavy chain variable region is most similar to the mouse heavy chain variable region subgroup "Miscellaneous" with a 73.3 % identity. This data suggests that the cDNAs cloned for the mouse 15 light and heavy chain variable regions do code for typical, functional mouse variable regions.

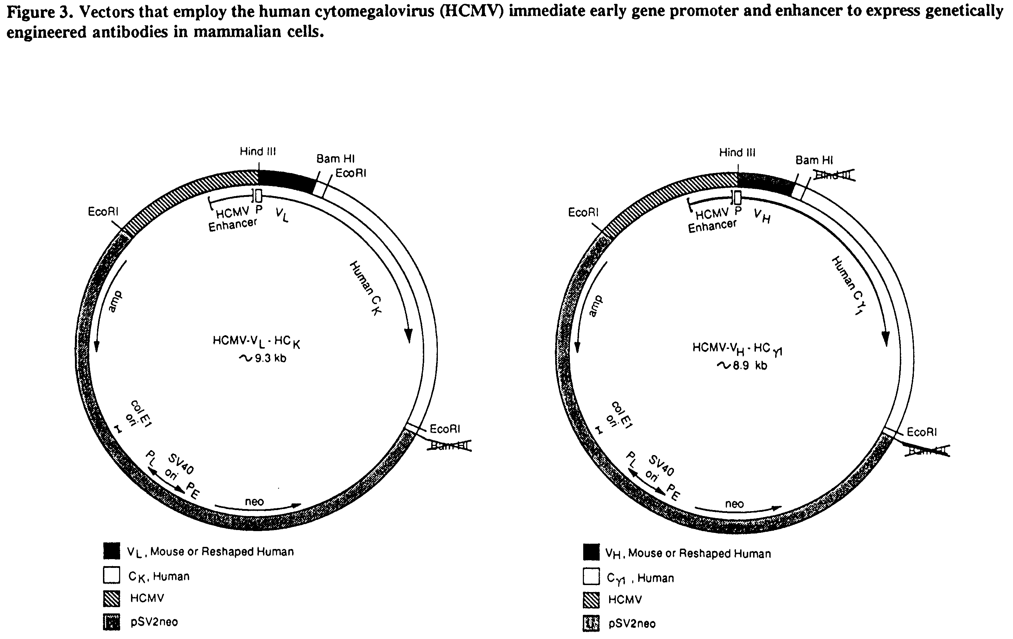

**Construction of chimeric 15 antibody.** Chimeric 15 antibody was constructed by linking the PCR-cloned mouse 15 light and heavy chain variable regions to human kappa and gamma-1 constant regions, respectively. Avaiable mammalian cell expression vectors already containing the necessary human constant regions were used (Figure 3). Both the light and heavy chain expression vectors are based on pSV2neo (Southern and Berg, 1982) and contain the human cytomegalovirus (HCMV) promoter and enhancer for efficient transcription of the immunoglobulin (IgG) light and heavy chains. These vectors contain a colE1 origin of replication for DNA replication in bacterial cells and a SV40 origin of replication for DNA replication and subsequent protein expression in mammalian cos cells. These vectors also contain unique HindIII and BamHI restriction enzyme sites used in the subcloning of the mouse 15 light and heavy chain leader and variable region DNA sequences into the HCMV vectors in the correct orientation (Figure 3).

The 5-' and 3'-ends of the mouse light and heavy chain variable regions were modified by PCR technology to enable efficient cloning and subsequent expression in the HCMV expression vectors. Modifications at the 5'-end included: (i) a Kozak sequence for efficient translation (Kozak, 1987), (ii) a HindIII restriction site for cloning into the HCMV vectors. Modifications at the 3'-end included: (i) a splice donor site at the J-C junction for splicing the mouse variable regions to the human constant regions, (ii) a BamHI restriction site for cloning into the HCMV vectors. The PCR primers designed for use in chimeric 15 antibody construction are shown in Table 3.

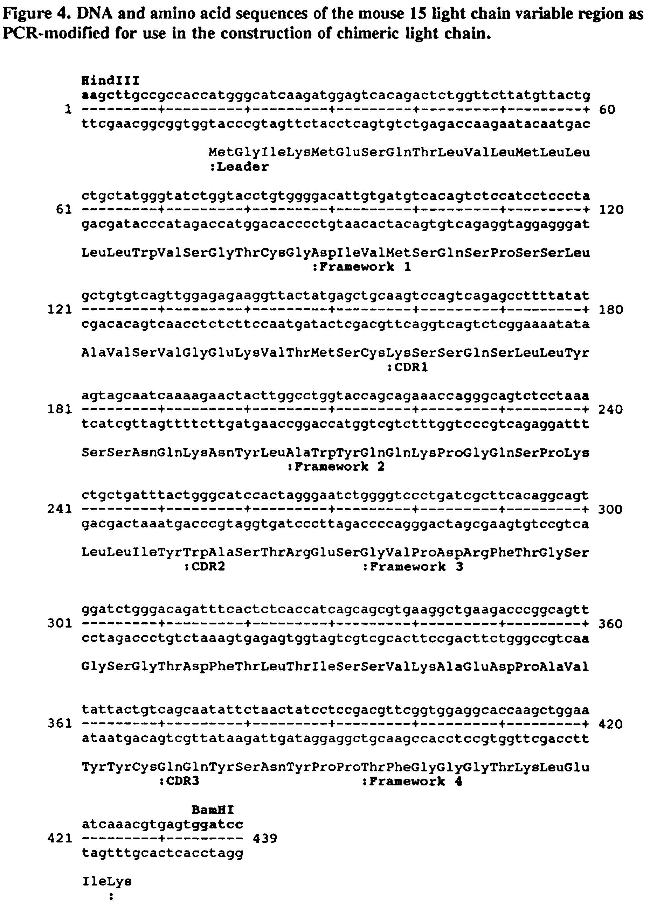

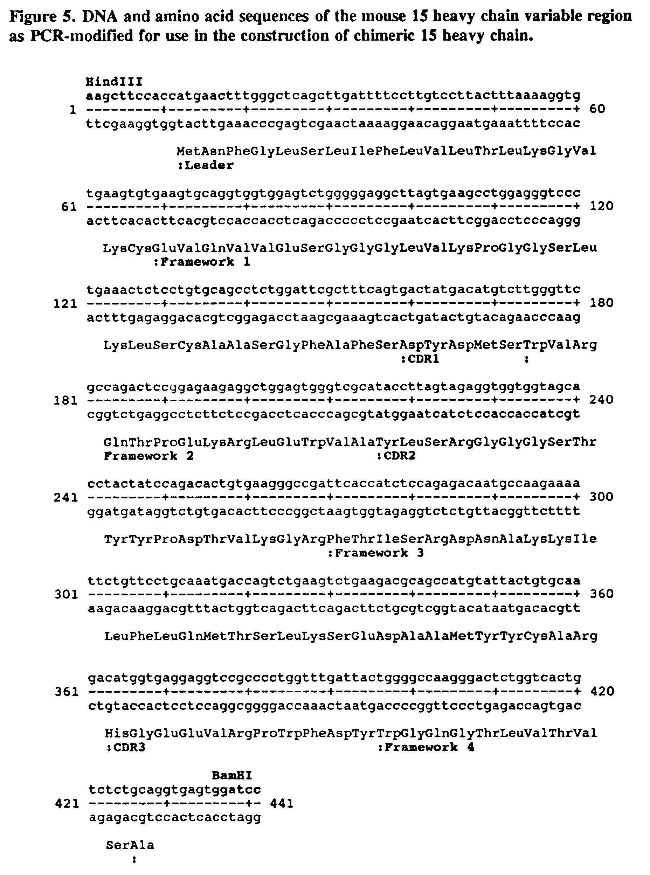

After PCR cloning using in chimeric primers, the products were re-sequenced to ensure that no errors were incorporated during PCR amplification. The DNA and amino acid sequences of the mouse 15 light and heavy chain variable regions as modified for the expression of chimeric 15 antibody are shown in Figures 4 and 5, respectively.

**Expression and analysis of chimeric 15 antibody.** The chimeric 15 light and heavy chain HCMV vectors were co-transfected, in duplicate, into mammalian cos cells. After 3 days of transient expression, the cell culture supernatants were collected. The supernatants were analyzed by ELISA to determine if human antibody was being produced. Good production of human IgG1 was observed (approximately 500 ng/ml). The supernatants were also tested in a cell-based ELISA for binding to human A431 cells which express the antigen recognized by MAb 15. The unpurified chimeric 15 antibody produced in the cos cells showed binding to the A431 cells. Purified mouse 15 antibody used as a standard also bound well to the same A431-coated plates indicating that the cell-based ELISA was functioning properly. In these early studies, the binding of the samples of mouse and chimeric 15 antibodies to A431 cells could not be directly compared because the antibody-enzyme conjugates used for detection are different for mouse and chimeric antibodies (see Methods). Supernatant from cos cells mock-transferred with no DNA showed no binding to A431 cells. A negative control with an anti-CMV glycoprotein antibody also gave negative results. Mouse 425 antibody served as an indirect positive control and gave good binding to A431 cells.

The unpurified chimeric 15 antibody produced in cos were analyzed for binding to unfixed TKB-2 target cells by direct immunofluorescence. The results indicated that the chimeric 15 antibody was comparable to mouse 15 antibody in binding to TKB-2 cells.

These early studies showed that chimeric 15 antibody, as expressed in mammalian cos cells, binds to the MAb 15 target cells in a manner similar to that of mouse 15 antibody. These results confirm that the correct mouse 15 light and heavy chain variable regions had been cloned and sequenced. The chimeric 15 antibody would also serve as an important control in the evaluation of the different versions of fully humanized, or reshaped human, 15 antibodies.

**Construction of a model of the structure of the mouse 15 variable regions.** A molecular model of the mouse variable regions of MAb 15 was constructed using the software package QUANTA (Polygen Corp.) running on a Silicon Graphics IRIS 4D. The framework regions for the light and heavy chain variable regions of MAb 15 were based on the structures of the mouse antibodies MCPC603 (Satow et al., 1987) and J529 (Suh et al., 1986), respectively. The L1, L2 and L3 CDR regions corresponded to the canonical structures proposed by Chothia et al. (1989), L1 being in Group 3 and L2 and L3 being in Group 1 of their relevant canonical structures (see Table 4). The H1 and H2 CDR regions also corresponded to canonical structures proposed by Chothia et al. (1989), H1 being in Group 1 and H2 being in Group 3 (see Table 5). The H3 CDR was modeled by searching a database of high resolution protein structures. The anchor points were C96, A97, R98 in framework region 3, and W110, G111, Q 112 in framework region 4. The best match was found in the heavy chain structure of MCPC603 in the region of CDR H3. The model was subjected to steepest descents and conjugate gradient energy minimization as implemented in QUANTA to relieve steric clashes.

**The design of reshaped human 15 antibody.** The first stage in the design process was to select the human light and heavy chain framework regions that would best serve as the basis of reshaped human 15 variable regions. The amino acid sequences of mouse 15 light and heavy chain variable regions (full-length variable regions and the framework regions only) were compared with the amino acid sequences of the consensus sequences for human subgroups as defined by Kabat et al. (1987) using the GAP program previously described. The comparisons are shown in Tables 6 and 7.

Generally, light chain variable region are less diverse than heavy chain variable regions and the light chain variable regions are thought to contribute less to the specificity of binding to antigen (Amit et al., 1986). The amino acid sequences of the mouse 15 light chain variable region show a high homology to all human kappa chain consensus subgroups (Table 6A). The mouse 15 light chain framework regions have a 82.5 % identity to human kappa subgroups 4 framework regions and a 75.0 % identity to human kappa subgroup 1 framework regions (Table 6A). A human kappa subgroup 1 antibody, human REI has been used many times previously as the basis for designing and constructing successful reshaped human light chain variable regions (for example, Riechmann et al., 1988; Kettleborough et al., 1991). Within the framework regions, the mouse 15 light chain variable region and the human REI variable region have a 75.0 % identity (Table 6B). An alignment of the framework regions of the mouse 15 and human REI light chain variable regions is shown in Table 6B. The REI frameworks as used in the construction of reshaped human 425 light chain variable region (Kettleborough et al., 1991 are well known. The human REI framework regions were thus selected as the basis for the design of the reshaped human 15 light chain variable region.

The amino acid sequences of the mouse 15 heavy chain framework regions were most similar to those of human heavy chain subgroups 3 with a 78.2 % identity (Table 7A). The reshaped human 15 heavy chain variable region has to be synthesised using synthetic DNA oligonucleotides according to standard techniques. In order to select an individual human antibody with a heavy chain variable region with the best fit to mouse 15 heavy chain variable region, the amino acid sequences of mouse 15 heavy chain variable regions were compared to all known human heavy chain variable regions as found in the LEEDS database of protein sequences. A human antibody was identified, human 30P1 antibody from human fetal liver (Schroeder et al. 1987). Within the framework regions of the heavy chain variable regions, there was 88.5 % similarity and 77.0 % identity between mouse 15 antibody and human 30P1 antibody. Human 30P1 antibody also has a published DNA leader sequence that was useful in the design of reshaped human 15 heavy chain variable regions. An alignment of the framework regions of the mouse 15 and human 30P1 heavy chain variable regions is shown in Table 7B. The human 30P1 framework regions were selected as the basis for the design of the reshaped human 15 heavy chain variable region.

The second stage in the design process was simply to join the CDR sequences from mouse 15 light and heavy chain variable regions to the framework region sequences from the selected human light and heavy chain variable regions. The mouse 15 light chain CDRs were joined to the framework regions from human REI light chain variable region as modified for use in reshaped human CAMPATH-1 and 425 antibodies. The mouse 15 heavy chain CDRs were joined to the framework regions from human 30P1 heavy chain variable region.

The third, and most complicated, stage in the design process was to identify and analyze the individual amino acid residues that differ between the sequences of the mouse 15 framework regions and the framework regions form the selected human antibodies. It is important to assess the role of the differing residues in determining the structure of the antibody variable regions and their subsequent influence, if any, on the antigen-binding site. The framework regions should remain as human as possible to reduce possible immunogenic reactions in patients and yet contain any amino acid residues present in the mouse sequences that are critical for binding to antigen.

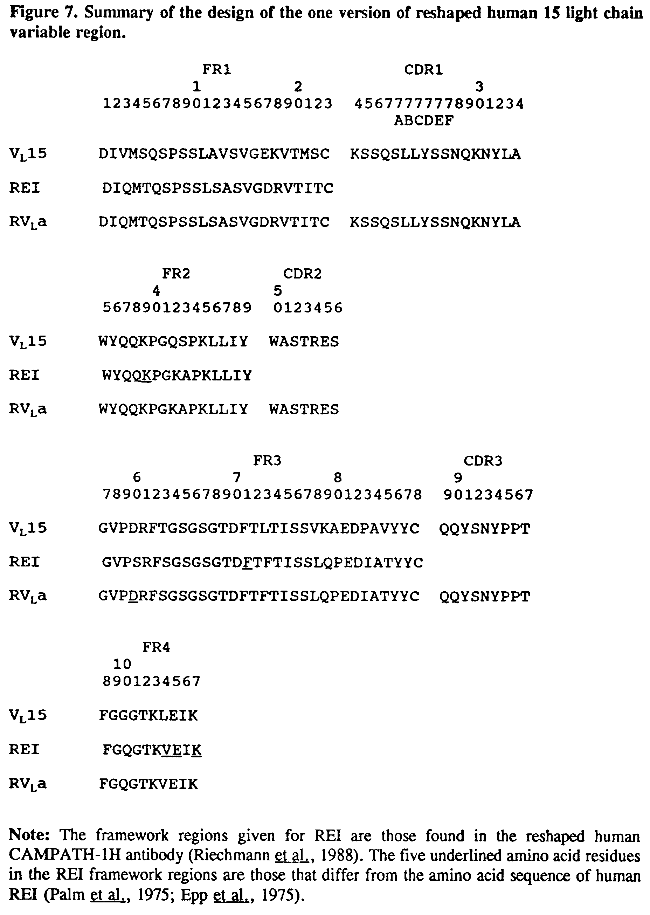

In designing the reshaped human 15 light chain variable region, the framework residues from human REI antibody and mouse 15 antibody were aligned and compared to each other as well as to the consensus sequences of the relevant subgroups of mouse and human kappa chain variable regions (Table 8). Within the framework regions, there are twenty-three amino acid residues that differ between mouse 15 and human REI light chain variable regions (Table 8). Between mouse 15 light chain variable region and the human REI light chain variable region as modified for use in CAMPATH-1H, there are twenty differences in the amino acid residues within the framework regions (Figure 7, Riechmann et al., 1988). Within the framework regions of human REI as modified for use in CAMPATH-1H, there are five differences from the original human REI (positions 39, 71, 104, 105, and 107 according to Kabat et al., 1987). The three changes in FR4 (position 104, 105, and 107) were based on a more typical human J region from another human kappa light chain and, therefore, do not constitute a deviation from human (Riechmann et al., 1988). The reshaped human 425 light chain variable region that was to serve as the template DNA for the construction of reshaped human 15 light chain variable region was constructed based on reshaped human CAMPATH-1 light chain variable region (Kettleborough et al. 1991).

Following evaluation of the twenty-three amino acid residues that differ between the framework region sequences of mouse 15 and human REI light chain variable regions, six residues were selected for change. Three of the residues are the three residues in FR4 (position 104, 105, and 107) that were modified in the construction of reshaped human CAMPATH-1 antibody. As explained above, these three changes were made based on the use of a more typical human J region (refer to FR4 of the consensus sequence for human kappa chain variable regions of subgroup 1, Table 8). These three changes do not constitute a deviation from human but instead are an improvement to a more typical human amino acid sequence in framework 4. The remaining three positions that were changed are positions 39, 60, and 71 (Table 8). Position 39 was changed from a threonin as present in human REI to a lysine as present in mouse 15, as well as in reshaped human CAMPATH-1. Position 60 was changed from a serine as present in human REI to aspartic acid as present in mouse 15. From the model of the structure of the mouse 15 variable regions, it appeared that the aspartic acid at position 60 might interact with the arginine at position 54 in the CDR2 loop. For this reason, it was decided best to keep the mouse amino acid residue at position 60 in FR3. Position 71 was changed from a tyrosine as present in human REI to a phenylalanine as present in mouse 15. Residue 71 in the light chain variable region is part of the canonical structure for the CDR1 loop (Chothia et al., 1989). It is important, therefore, to retain the same amino acid at this position as is present in the mouse 15 antibody. A summary of the design of the reshaped human 15 light chain variable region is presented in Figure 7.

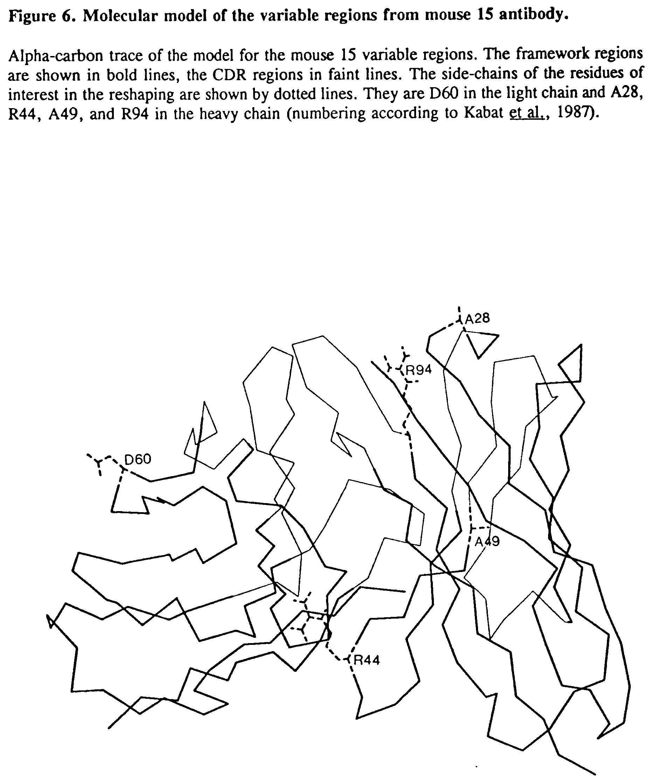

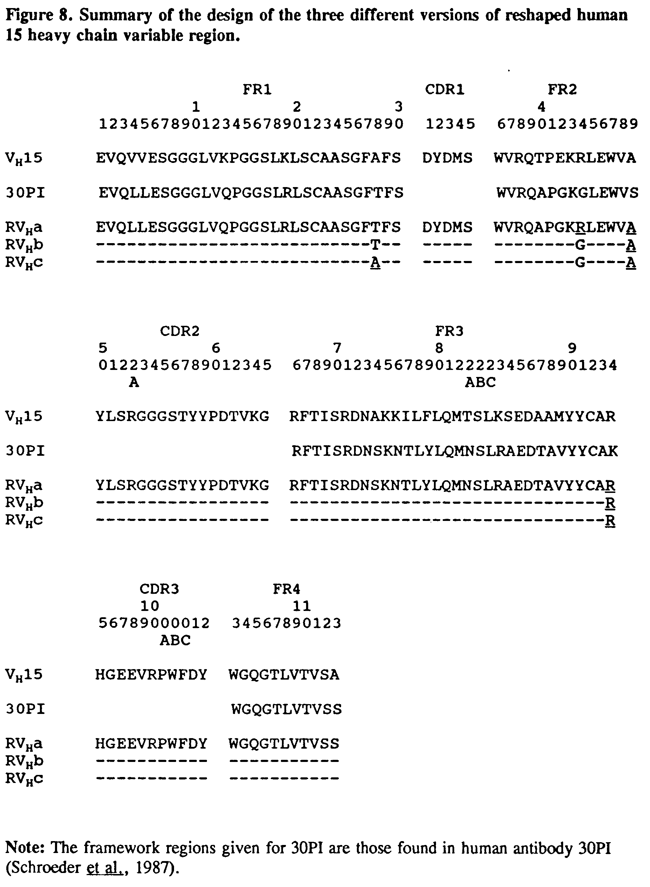

In designing the reshaped human 15 heavy chain variable region, the framework residues from human 30P1 antibody and mouse 15 antibody were aligned and compared to each other as well as to the consensus sequences of the relevant subgroups of mouse and human heavy chain variable regions (Table 9). Within the framework regions, there are twenty amino acid residues that differ between mouse 15 and human 30P1 heavy chain variable regions (Table 9). Following evaluation of the amino acid residues that differ between the framework region sequences of mouse 15 and human 30P1 heavy chain variable regions, four residues were selected for change, position 28, 44, 49, and 94 (Table 9). In version C of the reshaped human 15 heavy chain variable region, position 28 was changed from a threonine as present in human 30P1 to an alanine as present in mouse 15. Residue 28 in the heavy chain variable region is part of the canonical structure for the CDR1 loop (Chothia et al., 1989). As seen in the model of the structure of the mouse 15 variable regions, residue 28 is part of the structural H1 loop that probably interacts directly with antigen (Figure 6). It is important, therefore, to retain the same amino acid at this position as is present in the mouse 15 antibody. In version A of the reshaped human 15 heavy chain variable region, position 44 was changed from a glycine as present in human 30P1 to an arginine as present in mouse 15. Based on the structural model of the mouse 15 variable regions (Figure 6), residue 44 was thought to have a possible role in the packing of the mouse 15 light and heavy chain variable regions. Residue 44, however, is not one of the residues identified by Chothia et al. (1985) and Chothia and Lesk (1987) as having a role in V_{L}-V_{H} packing. In all three versions of reshaped human 15 heavy chain variable regions, position 49 was changed from a serine as present in human 30P1 to an alanine as present in mouse 15. Residue 49 is abutting the CDR2 region and from the model of the structure of the mouse 15 variable regions, it appears that if the larger serine residue is inserted in place of the alanine at this position in the mouse 15 sequence, it may disrupt the conformation of the CDR2 loop (Figure 6). In all three versions of reshaped human 15 heavy chain variable regions, position 94 was changed from a lysine as present in human 30P1 to an arginine as present in mouse 15. Residue 94 in the heavy chain variable region is part of the canonical structure for the CDR1 loop (Chothia et al., 1989). The model of the structure of the mouse 15 variable regions shows that residue 94 is important in the packaging of the CDR1 loop, and possibly the CDR3 loop, against the framework residues. Again, it is important that residues that are identified as belonging to a canonical structure for loop conformation be kept the same amino acid as is present in the mouse 15 antibody. A summary of the design of the three versions of reshaped human 15 heavy chain variable regions is presented in Figure 8.

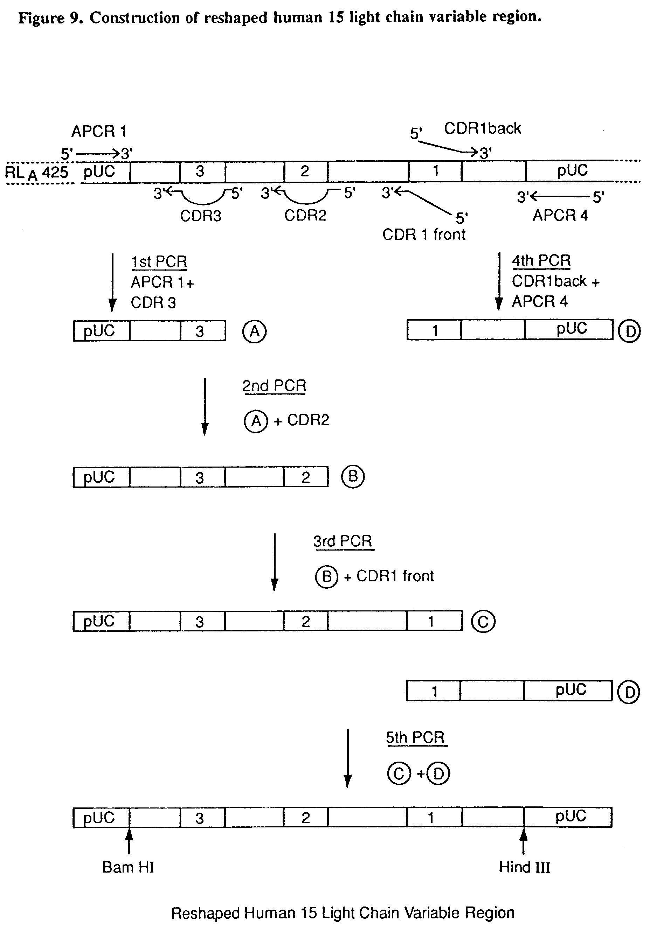

**Construction of the reshaped human 15 variable regions.** The construction of the reshaped human 15 light chain was carried out using a novel method that was based on PCR-mutagenesis methods (Kamman et al., 1989). The CDRs form mouse 15 light chain variable regions were sequentially grafted onto the human REI frameworks (see Methods and Figure 9). The DNA template used was reshaped human 425 light chain variable region version a (Kettleborough et al., 1991) which was cloned as a HindIII-BamHI fragment into a pUC18 vector. Four synthetic oligonucleotides were designed and synthesized that linked the CDR coding sequences from mouse 15 light chain variable regions to the framework regions of reshaped human 425 light chain variable region (Table 10). The change at position 60 in FR3 from the human serine to the mouse aspartic acid was incorporated in the CDR2-grafting primer (Table 10). The CDR1 of mouse 15 light chain variable region was incorporated using back and front oligonucleotides of approximately 50 bp (Table 10). These two primers hybridize within FR1 and FR2 of reshaped human 425 light chain variable region, respectively (Figure 9). The two primers overlap and hybridize to each other within 19 bp of the mouse CDR1. The construction method also required two external PCR primers that flank the full-length reshaped human 15 light chain variable region (Table 10, Figure 9). Once the full-length reshaped human 15 light chain variable region was constructed, it was cloned into a pUC19 vector and sequenced. The DNA sequence of four clones showed that they all contained PCR-induced errors, both deletions and point mutations. The reshaped human 15 light chain variable region from one clone that had only two point mutations that did not alter the amino acid sequence, positions 422 (C-T) and 479 (G-A), was subcloned into the mammalian HCMV expression vector containing the human constant region (Figure 3). The DNA and amino acid sequences of the reshaped human 15 light chain variable region are shown in Figure 10.

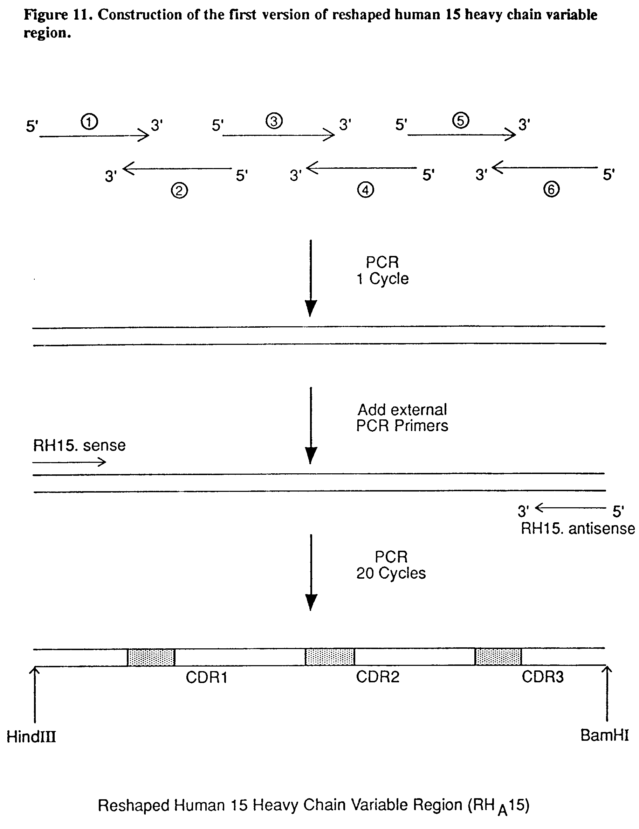

The first version of reshaped human 15 heavy chain variable region was completely constructed from synthetic DNA oligonucleotides (Figure 11). The DNA sequence to be constructed was designed based on the DNA sequence of the human 30P1 leader and heavy chain variable region and the DNA sequences of the CDRs from mouse 15 heavy chain variable region. The full-length reshaped human 15 heavy chain variable region was assembled by adapted PCR methods (Higuchi et al., 1988; Lewis et al., 1991; and Daugherty et al., 1991). Six synthetic oligonucleotides were designed to span the full-length of the reshaped human 15 heavy chain region (Table 11) and PCR-assembled as illustrated in Figure 11. Three sense DNA oligonucleotides (1, 3, and 5) and three anti-sense oligonucleotides (2, 4, and 6) were annealed and extended for 1 PCR cycle. The full-length product was then amplified for 20 cycles using the external forward and reverse PCR primers (Table 11). Once the full-length reshaped human 15 heavy chain variable region was PCR assembled, the DNA product was cloned into a pUC19 vector for sequencing. Of the four DNA clones sequenced, one had the correct full-length sequence with no point mutations or deletions. This positive clone was subcloned into the HCMV expression vector containing the human heavy chain gamma-1 constant region (Figure 3). The DNA and amino acid sequences of reshaped human 15 heavy chain variable region (version A) are shown in Figure 12.

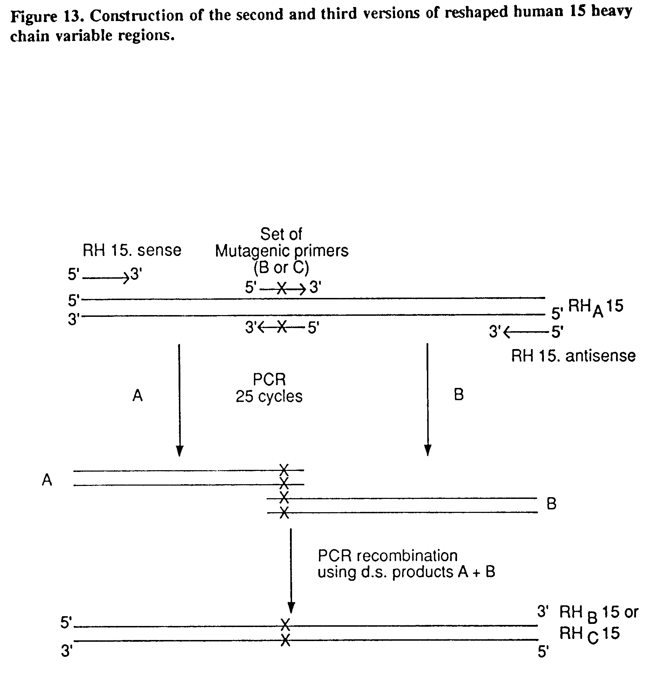

The subsequent two versions of reshaped human 15 heavy chain variable region (versions B and C) were constructed using modified recombinant PCR-mutagenesis techniques (Higuchi et al., 1988). Version C was constructed as shown in Figure 13. Two overlapping, complementary mutagenic primers of 20 bps each were designed and synthesized (Table 12). Both primers contained a one base-pair mismatch to introduce the threonine to alanine change at position 28 (Table 9). Primary PCR reactions were carried out using the two external PCR primers with the two mutagenic PCR primers (Table 12 and Figure 13). The two primary PCR products were then combined in a PCR reaction and the resulting full-length product was amplified using the external PCR primers (Figure 13). After subcloning into a pUC19 vector, four clones were DNA sequenced. Three of the clones had the desired mutation introduced. The construction of version B was similar to that of version C except that a unique BanI restriction enzyme site was present in the region of overlap and this site was used to ligate the two primary PCR products together. The two mutagenic PCR primers used to construct version B had an overlap of 14 bps (Table 12). The final DNA products were cloned into a pUC19 vector and two clones were sequenced. One of the clones contained the correctly mutated DNA sequence.

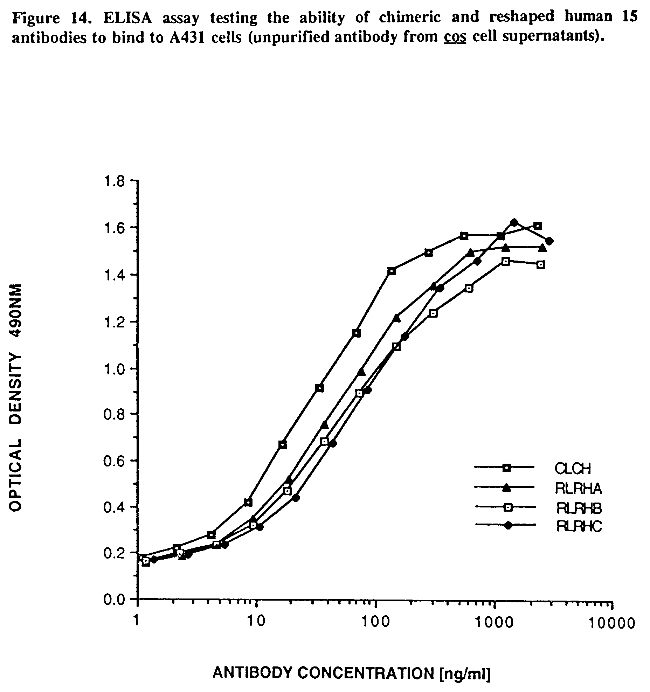

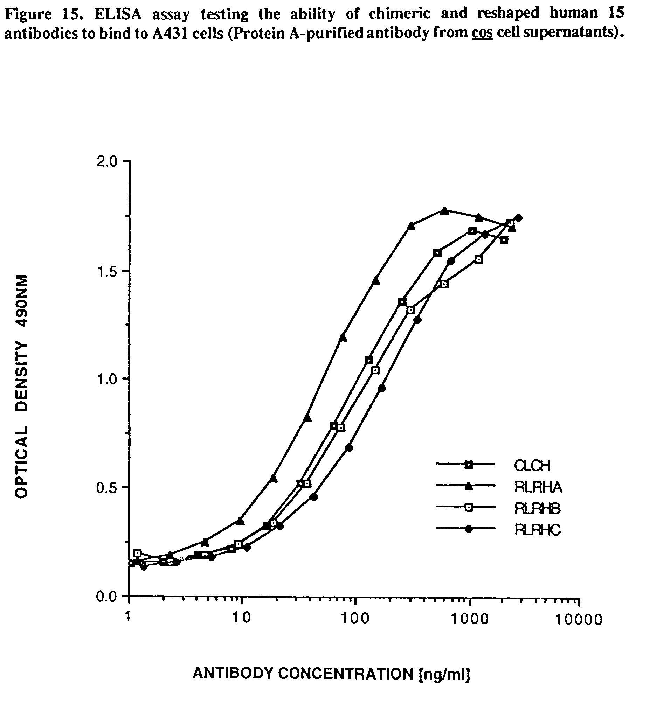

**Expression and analysis of the different versions of reshaped human 15 antibody.** The one version of reshaped human 15 light chain and the three versions of reshaped human 15 heavy chain were transiently co-expressed in pairs in the mammalian cell line cos. The chimeric and reshaped human 15 antibodies were produced in good amounts in cos cells (between 1.5 to 2.0 µg/ml as quantified by ELISA). Initially, unpurified samples of antibody as present in cos cell supernatants were analyzed for binding to the A431 target cells (Figure 14). From the binding curves obtained for the chimeric and for the different versions of reshaped human 15 antibody, it appeared that chimeric antibody was slightly better than the three versions of reshaped human antibodies and that of the three versions of reshaped human 15 antibody, version L+Ha was best. The chimeric and reshaped human 15 antibodies were Protein A-purified from cos cell supernatants and re-analyzed for binding to A431 cells (Figure 15). Within the samples of purified antibody, version L+Ha of reshaped human 15 antibody was binding to cells slightly better than the chimeric 15 antibody. Versions L+Hb and L+Hc of reshaped human 15 antibody were binding almost as well as chimeric 15 antibody.

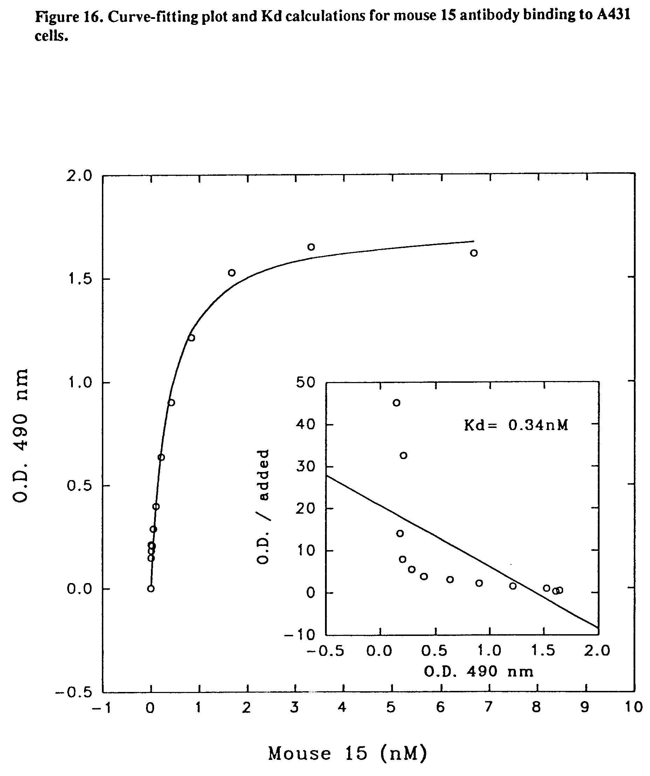

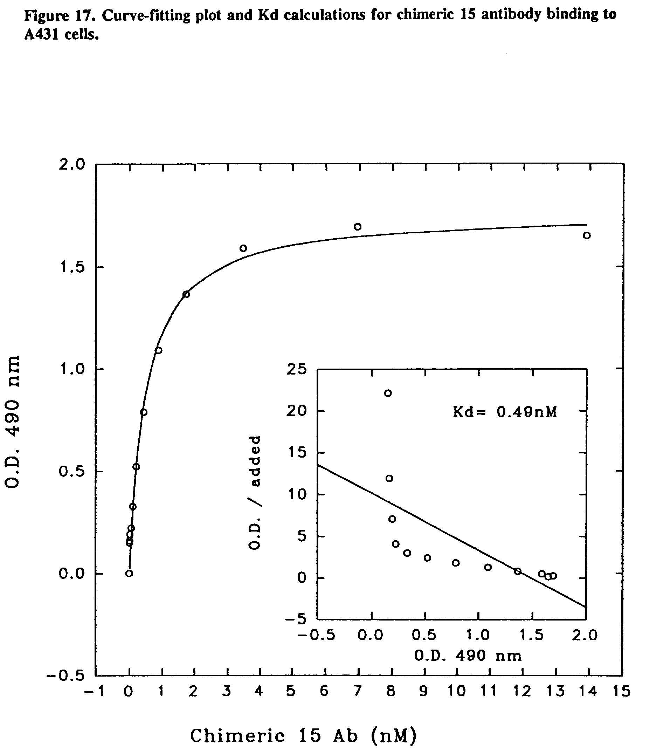

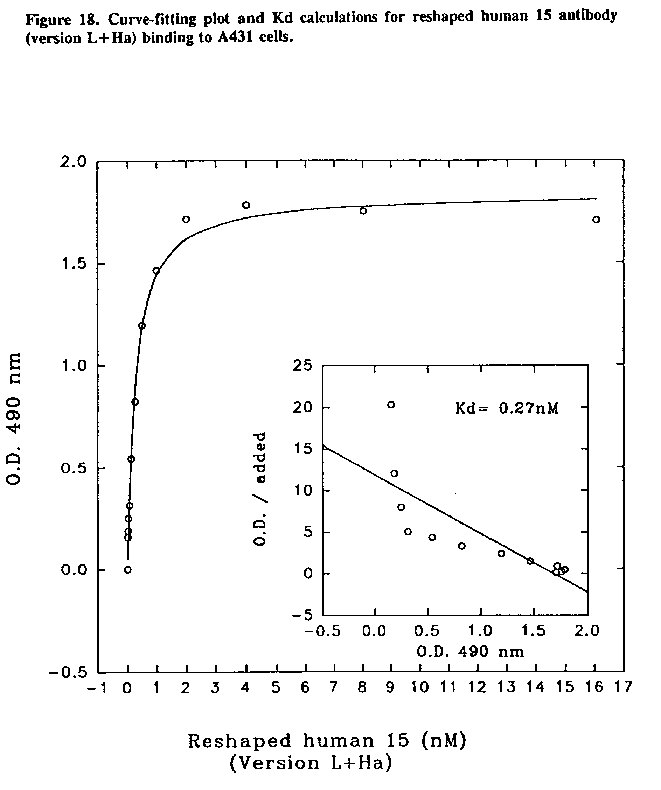

**Determination of the affinity constants for mouse, chimeric, and reshaped human 15 antibodies.** The antigen affinities of the mouse, chimeric, and version L+Ha of reshaped human 15 antibodies were determined using a sample of purified mouse 15 antibody and the same samples of Protein A-purified chimeric and reshaped human 15 antibodies that were used to obtain the data shown in Figure 15. The data from the ELISA assays were fitted to the best curve using SIGMA PLOT and the concentrations of antibody that gave 50 % saturation were determined. The results are shown in Figures 16, 17, and 18. The Kds determined were 0.34 nM for mouse 15 antibody, 0.49 nm for chimeric 15 antibody, and 0.27 nm for reshaped human 15 antibody (version L+Ha). Although this relatively simple method of determining the affinities based on an ELISA assay can not be expected to yield the most definitive measurements of affinity, these results indicate that the original mouse 15 antibody and the reshaped human 15 antibody (version L+Ha) bind well to the A431 cells. In addition, the binding by reshaped human 15 antibody (version L+Ha) may be slightly better than the binding by the mouse 15 antibody. In summary, the best version of reshaped human 15 antibody has an affinity for antigen that is approximately equal to that of mouse 15 antibody or chimeric 15 antibody.

The mouse 15 light and heavy chain variable regions were successfully PCR cloned from the mouse MAb 15 hybridoma cell line using the PCR primers designed by Jones and Bendig (1991). For the mouse 15 heavy chain variable regions, the DNA sequences from two independent PCR products were identical and the amino acid translation gave a full-length mouse heavy chain variable region (Figure 2). For the mouse 15 light chain variable region, two different light chain variable regions were PCR cloned. DNA sequencing and subsequent amino acid translation showed that only one was a full-length variable region with no stop codons present (Figure 1). The second light chain variable region was identified as a non-functional mopc21n kappa chain probably derived from the myeloma cell line P3X63Ag8U.1 than was used as a fusion partner during the construction of the mouse MAb 15 hybridoma cell line (Endo et al. 1986). Comparison with the consensus sequences for the different groups of mouse light and heavy chain variable regions indicated that the mouse 15 variable regions were typical mouse sequences (Table 2).

Confirmation that the cloned mouse light and heavy chain variable regions were those of mouse 15 antibody was obtained by constructing and expressing chimeric 15 antibody. The mouse 15 variable regions were linked to human constant regions and inserted into mammalian cell expression vectors. Unpurified chimeric 15 antibody produced in cos cells showed good binding to human A431 in a cell-based ELISA assay. Unpurified samples of chimeric antibody also showed good binding to TKB-2 cells. These results confirmed that the correct mouse 15 sequences had been cloned. The chimeric 15 antibody was subsequently used as a positive control to access the antigen binding of the different versions of reshaped human 15 antibody.

The reshaped human 15 variable regions were designed by first selecting human frameworks that had a high percent identity to the mouse 15 frameworks. In the case of the light chain, the human REI frameworks were chosen. For the heavy chain, the human 30P1 framework regions were chosen. A model of the structure of the mouse 15 variable regions was built based on homology to antibodies with solved structures (Figure 6). Analysis of the model and available information on the conformations of immunoglobulin variable regions were used to identify amino acid positions in the framework regions that should be kept as the same amino acid present in mouse 15 antibody. It is important at this stage to keep the reshaped antibody as human as possible and select only those residues that are critical in determining the conformation of the hypervariable loops and thus in creating a good antigen-binding site.

One version of the reshaped human 15 light chain variable region was designed and constructed. It contained only three changes in the framework regions that differ from the sequences of known human antibodies (Table 8, Figure 7). Three versions of the reshaped human 15 heavy chain variable regions were designed and constructed. The best version, version A, contained only three changes in the framework regions that differed from the sequence of the known human antibody 30P1 (Table 9, Figure 8).

The construction of the reshaped human 15 light chain variable region by sequentially grafting the mouse 15 CDRs onto the human REI framework regions using a PCR-based method had a high frequency of PCR-induced errors. Probably due to the number of PCR steps involved, all of the clones sequenced had mutations present. The construction of the reshaped human 15 heavy chain variable region by PCR assembly (Figure 13) was more straight-forward and a lower frequency of PCR-induced errors was observed. In this case, the second method was the preferred method for constructing a reshaped human variable region.

The mouse, chimeric, and reshaped human 15 antibodies were tested in ELISA assays for binding to human A431 cells. It was not possible to make a direct comparisons between the binding curves obtained for mouse antibody and those obtained for chimeric or reshaped human antibodies because the antibodies used for detection were different. The mouse antibody was detected with a goat anti-mouse antibody and the chimeric and reshaped human antibodies were detected with a goat anti-human antibody. It was possible, however, to directly compare the binding curves for chimeric and reshaped human antibodies. A chimeric antibody is expected to bind to antigen in a manner identical to that seen with the original mouse antibody. Numerous ELISA assays indicated that of the three reshaped human 15 antibodies, version A of the heavy chain was slightly better than versions B or C. Most importantly, this best reshaped human 15 antibody bound to antigen as well as chimeric 15 antibody. In some assays, the chimeric 15 antibody was slightly better and in other assays the reshaped human 15 antibody was slightly better. Within the accuracy that can be achieved with this type of ELISA assay, the reshaped human 15 antibody with version A of the heavy chain is considered to have a good affinity for antigen that is essentially equal to that of the chimeric 15 antibody.

Version A of reshaped human 15 heavy chain variable region appeared to create the best reshaped human 15 antibody. The mouse residue at position 44 appeared to slightly improve the binding of the reshaped human 15 antibody. From the model, residue 44 appears to be at the V_{L}-V_{H} interface and may be involved in packing of the V_{L} and V_{H} domains. Surprisingly, residue 44 is not a conserved packing residue as defined by Chothia et al. (1985 and 1987). Residue 28 was also analyzed for its influence on antigen binding. Residue 28 is part of the canonical structure of the CDR1 loop in the heavy chain variable region (Chothia et al., 1989). Comparing the results with versions B and C, it appears that the differences in binding to A431 cells are negligible. Surprisingly, in this antibody, residue 28 in the heavy chain variable region does not appear to be at a very critical position for determining binding to antigen.

In order to more carefully compare and estimate the affinities of the mouse 15 antibody, the chimeric 15 antibody, and the best version of reshaped human 15 antibody, purified and concentrated samples of the antibodies were tested, in duplicate and over a broad range of dilutions, in the ELISA assay for binding to A431 cells. These data were analyzed using a plotting program that fitted the data to a saturation-binding curve and then determined the disassociation constant (Kd) by calculating the free antibody concentration at half-saturation of the antigen binding sites. For mouse, chimeric, and reshaped human 15 antibodies the Kds were 0.34 nM, 0.49 nM, and 0.27 nM, respectively, thus indicating that the reshaping process has yielded a reshaped human 15 antibody that binds to cell with an affinity that is at least equal to that of mouse or chimeric 15 antibody.

A more careful evaluation of the best version of reshaped human 15 antibody is now needed. One suggestion is to carry out competition binding assays between the unlabelled mouse, chimeric, and reshaped human 15 antibodies and the labelled mouse 15 antibody. Another possibility to consider is attempting to get accurate measurements of antigen affinity using the biosensor machine (BIAcore) recently introduced by Pharmacia. This would require samples of antigen that could be attached to dextran on the gold film in the BIAcore machine. The in-depth analysis of reshaped human 15 antibody will require larger amounts of purified antibody. The DNA coding for the reshaped human 15 heavy chain has been introduced into a dhfr gene amplification vector. This vector and the vector that expresses the reshaped human 15 light chain have been co-transfected into CHO dhfr cells. Following the initial selection for cells containing the two vectors, the production of antibody will be increased by selecting in methotrexate for gene amplification.

### Therapeutic and diagnostic use

The antibody fragments and whole antibodies according to the invention can be administered to human patients for therapy. Therefore, it is an object of the invention to provide a pharmaceutical formulation comprising as active ingredient at least one antibody or antibody fragment as defined above and in the claims, associated with one or more pharmaceutically acceptable carrier, excipient or diluent therefore.

Typically the antibody of this invention will be injected intravenously or parenterally. Generally, the dosage ranges for the administration of the antibodies fragments are large enough to produce the desired tumor suppressing and tumor lysing effect. The dosage will depend on age, condition, sex and extent of the disease in the patient and can vary from 0.1 mg/kg to 200 mg/kg, preferably from 0.1 mg/kg to 100 mg/kg/dose in one or more doses administered daily, for one or several days.

Preparations for parenteral administration includes sterile aqueous or non-aqueous solutions, suspensions, and emulsions. Examples of non-aqueous solvents are propylene glycol, polyethylene glycol, vegetable oils such as olive oils, and injectable organic esters such as ethyl oleate and other solvents known in the art which are suitable for these purposes. The antibodies of this invention can be used in a composition comprising a physiologically acceptable carrier. Examples of such suitable carriers are saline, PBS, Ringer's solution, or lactated Ringer's solution. Preservatives and other additives such as antibiotics, antioxidants, and chelating agents may also be present in the pharmaceutical formulations.

The antibody according to the invention or a fragment thereof can also be conjugated according to known methods to cytokines such as IL-2 in order to support their cytotoxicity.

The pharmaceutical formulations of the present invention are suitable for the treatment of all kinds of tumors, including melanomas, gliomas and carcinomas, as well as tumors of the circulating system and especially solid tumors, preferrably, lung carcinoma.

For diagnostic purposes the antibody can be conjugated, for example, to a radio-opaque dye or can be radiolabelled. A preferred labelling method is the lodogen method. The antibody can also be administered as F(ab')₂ or scFv fragments for diagnostic purposes. This provides superior results so that backround substraction is unnecessary.

### REFERENCES

Amit, A.G., R.A. Mariuzza, S.E. Phillips, and R.J. Poljak. 1986. Three-dimensional structure of an antigen-antibody complex at 2.8Å resolution. Science **233**: 747-753.
Chirgwin, J.M., A.E. Przybyla, R.J. MacDonald, and W.J. Rutter. 1979.Isolation of biologically active ribonucleic acid from sources enriched in ribonuclease. Biochemistry **18**: 5294-5299.
Chothia, C. and A.M. Lesk. 1987. Canonical structures for the hypervariable regions of immunoglobulins. J. Mol. Biol. **196**: 901-907.
Chothia, C., A.M. Lesk, A. Tramontano, M. Levitt, S.J. Smith-Gill. Air, S. Sheriff, E.A. Padlan, D. Davies, W.R. Tulip, P.M. Colman, S. Spinelli, P.M. Alzari, and R.J. Poljak. 1989. Conformations of immunoglobulin hypervariable regions. Nature **342**: 877-883.
Chothia, C., J. Novotny, R. Bruccoleri, and M. Karplus. 1985. Domain association in immunoglobulin molecules: The packing of variable domains. J. Mol. Biol. **186**: 651-663.
Daugherty, B.L., J.A. Demartino, M.-F. Law, D.W. Kawka, I.I. Singer, and G.E. Mark. 1991. Polymerase chain reaction facilitates the cloning, CDR-grafting, and rapid expression of a murine monoclonal antibody directed against the CD18 component of leukocyte integrins. Nucleic Acis Res. **19**: 2471-2476.
Endo, K., H. Kamma, and T. Ogata. 1986. Two murine monoclonal antibodies against human lung cancer-associated antigens. Cancer Research **46**: 6369-6373.
Endo, K., H. Kamma, T. Ogata. 1988. Radiolabeled monoclonal antibody 15 and its fragments for localization and imaging of xenografts of human lung cancer. J. Nat. Cancer Institute **80**: 835-842.
Epp, O., P. Colman, H. Fehlhammer, W. Bode, M. Schiffer, R. Huber, and W. Palm. 1974. Crystal and molecular structure of a dimer composed of the variable portions of the Bence Jones protein REI. Eur. J. Biochem. **45**: 513-524.
Higuchi, R., B. Krummel, and R.K. Saiki. 1988. A general method of in vitro preparation and specific mutagenesis of DNA fragments: study of protein and DNA interactions. Nucleic Acids Res. **16**: 7451-7367.
Jones, S.T. and M.M. Bendig. 1991. Rapid PCR-cloning of full-length mouse immunoglobulin variable regions. Bio/Technology **9**: 88-89.
Kabat, E.A., T.T. Wu, M. Reid-Miller, H.M. Perry, and K.S. Gottesman. 1987. Sequences of Proteins of Immunological Interest, Fourth Edition, U.S. Department of Health and Human Services, U.S. Government Printing Office.
Kamma, H., K. Endo, H. Horiguchi, T. Iijima, and T. Ogato. 1989. Lung cancer-associated monoclonal antibody 15 that recognizes cell growth-related membrane antigens gp85/45 and its growth-inhibitory effect on human lung cancer cells in vitro. Cancer Research **49**: 5118-5112.
Kamman, M., J. Laufs, J. Schell, and B. Bronenborn, 1989. Rapid insertional mutagenesis of DNA by polymerase chain reaction (PCR). Nucl. Acids Res. **17**: 5404.
Kettleborough, C.A., J. Saldanha, V. Heath, C.J. Morrison, M.M. and Bendig 1991. Humanisation of a mouse monoclonal antibody by CDR-grafting: The importance of framework residues on loop conformation. Protein Engineering **4**: 773-783.
Khazaeli, M.B., M.N. Saleh, T.P. Liu, R.F. Meredith, R.H. Wheeler, T.S. Baker, D. King, D. Secher, L. Allen, K. Rodgers, D. Colcher, J. Schlom, D. Shochat, and A.F. LoBuglio. 1991. Cancer Res. **51**: 5461-5466.
Kozak, M. 1987. At least six nucleotides preceding the AUG initiator codon enhance translation in mammalian cells. J. Mol. Biol. **196**: 947-950.
Larrick, J.W., L. Danielson, C.A. Brenner, E.F. Wallace, M. Abrahamson, K.E. Fry, and C.A. Borrebaeck. 1989. Bio/Technology **7**: 934-938.
Lewis, A.P. and J.S. Crowe. 1991. Immunoglobulin complementarity-determining region grafting by recombinant polymerase chain reaction to generate humanised monoclonal antibodies. Gene **101**: 297-302.
LoBuglio, A.F., R.H. Wheeler, J. Trang, A. Haynes, K. Rogers, E.B. Harvey, L. Sun, J. Ghrayeb, and M.B. Khazaeli. 1989. Mouse/human chimeric monoclonal antibody in man: Kinetics and immune response. Proc. Natl. Acad. Sci. USA **86**: 4220-4224.
Meredith, R.F., A.F. LoBuglio, W.E. Plott, R.A. Orr, I.A. Brezovich, C.D. Russell, E.B. Harvey, M.V. Yester, A.J. Wagner, S.A. Spencer, R.H. Wheeler, M.N. Saleh, K.J. Rodgers, A. Polansky, M.M. Slater, M.B. Khazaeli. 1991. J. Nucl. Med. **32**: 1162-1128.
Palm, W., N., Hoppe-Seyler's. 1975. Physiol. Chem. **356**: 167-191.
Quacekbush, E., M. Clabby, K.M. Gottesdiener, J. Barbosa, N.H., J.L. Strominger, S. Speck, and J.M. Leiden. 1987. Molecular cloning of complementary DNAs encoding the heavy chain of the human 4F2 cell-surface antigen: A type II membrane glycoprotein involved in normal and neoplastic cell growth. Proc. Natl. Acad. U.S.A. **84**: 6526-6530.
Riechmann, L., M. Clark, H. Waldmann, and G. Winter. 1988. Reshaping human antibodies for therapy. Nature **332**: 323-327.
Saleh, M.N., M.B. Khazaeli, R.H. Wheeler, L. Allen, A.B. Tilden, W. Grizzle, R.A. Riesfeld, A.L. Yu, S.D. Gillies, and A.F. LoBuglio. 1992. Phase I trial of the chimeric anti-GD2 monoclonal antibody ch14.18 in patients with malignant melanoma. Human Antibod. Hybridomas **3**: 19-24.
Satow, Y., G.H. Cohen, E.A. Padlan, and D.R. Davies. 1987. Phosphocholine binding immunoglobulin FAB MCPC603. J. Mol. Biol. **190**: 593.
Schroeder, H.W., J.L. Hillson, and R.M. Perlmutter. 1987. Early restriction of the human antibody repertoire. Science **238**: 791-793.
Southern, P.J. and P. Berg. 1982. Transformation of mammalian cells to antibiotic resistance with a bacterial gene under the control of the SV40 early region promoter. J. Mol. Appl. Genet. **1**: 327-341.
Suh, S.W., T.N. Bhat, M.A. Navia, G.H. Cohen, D.N. Rao,S. Rudikoff, and D.R. Davies. 1986. FAB J539. Proteins **1**: 74.
Teixeira, S., S. DiGrandi, and L.C. Kühn. 1987. Primary structure of the human 4F2 antigen heavy chain predicts a transmambrane protein with a cytoplasmic NH₂ terminus. J. Biol. Chem. **262**: 9574-9580.
Walfield, A., E. Selsing, B. Arp, and U. Strob. 1981. Misalignment of V and J gene segments resulting in a nonfunctional immunoglobulin gene. Nucl. Acids Res. **9**: 1101-1109.

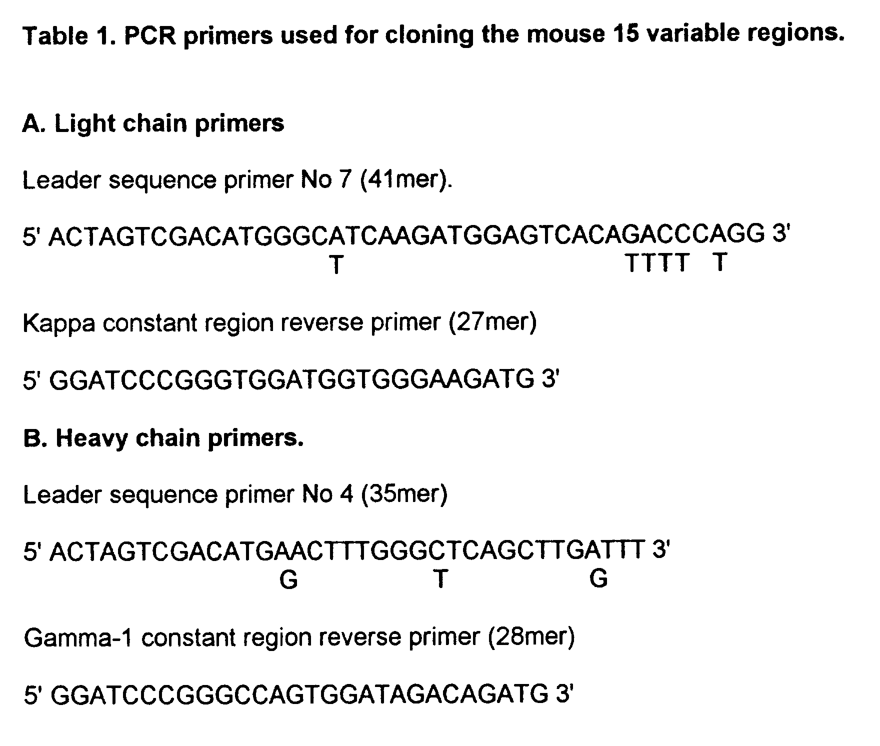

**Table 2**

| **Comparison of the mouse 15 variable regions to the consensus sequences for mouse variable regions.** **A. Light chain variable region.** | |
|---|---|
| Mouse kappa subgroup | Percent identity to MAb 15 |
| k1 | **72.566** |
| k2 | 68.750 |
| k3 | 64.865 |
| k4 | 65.741 |
| k5 | 65.421 |
| k6 | 62.264 |
| k7 | 39.394 |
| Mis. | 58.333 |

| **B. Heavy chain variable region.** | |
|---|---|
| Mouse kappa subgroup | Percent identity to MAb 15 |
| H1a | 51.667 |
| H1b | 53.333 |
| H2a | 48.333 |
| H2b | 45.833 |
| H2c | 50.000 |
| H3a | 61.345 |
| H3b | 60.000 |
| H3c | 62.609 |
| H5a | 46.667 |
| H5b | 44.737 |
| Mis. | **73.333** |

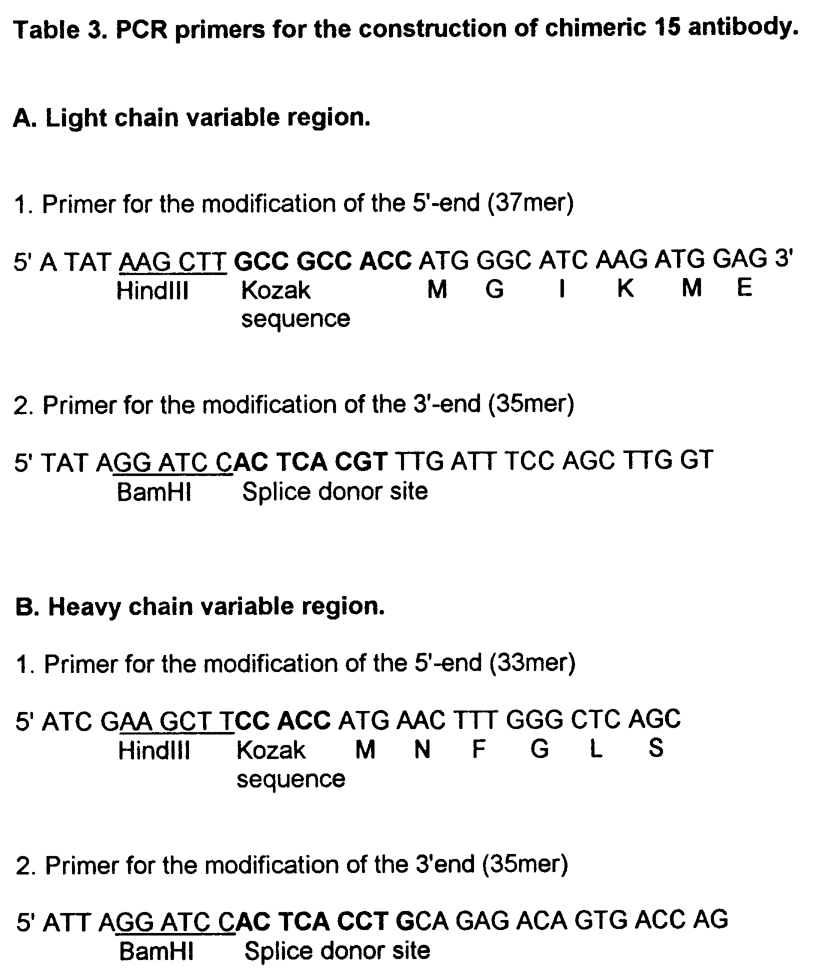

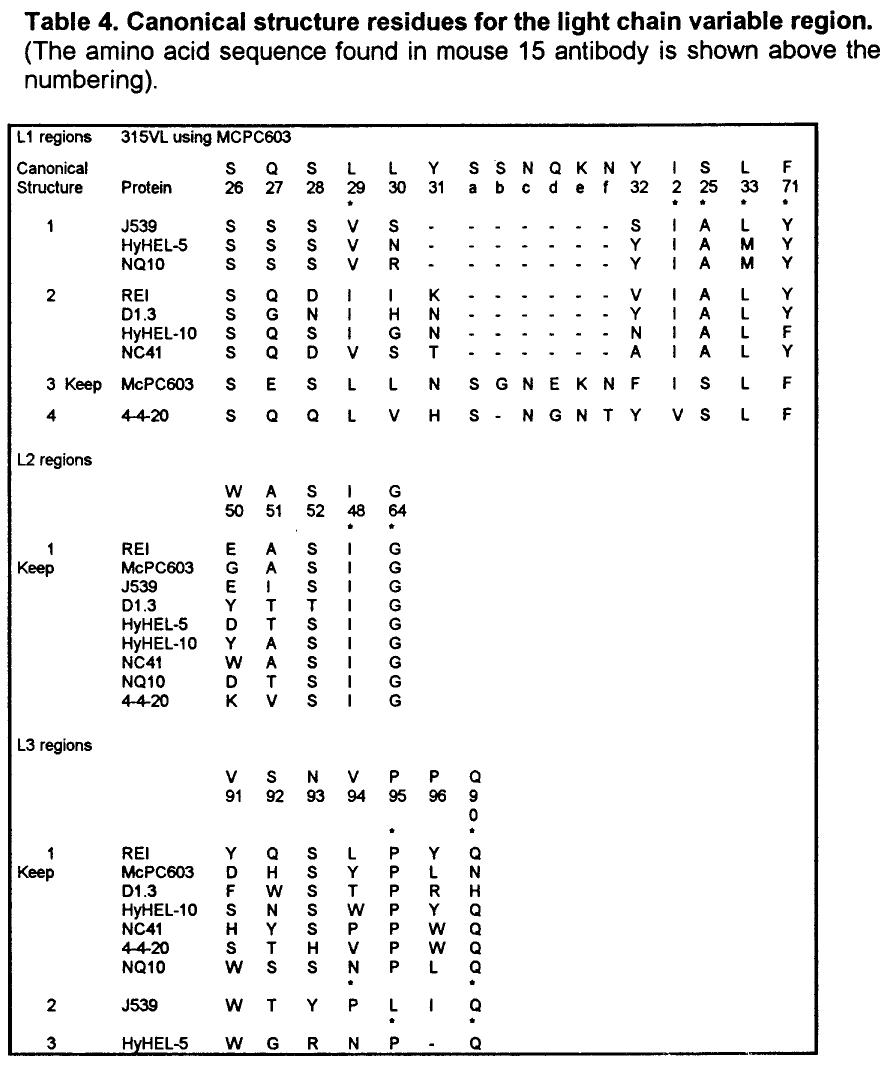

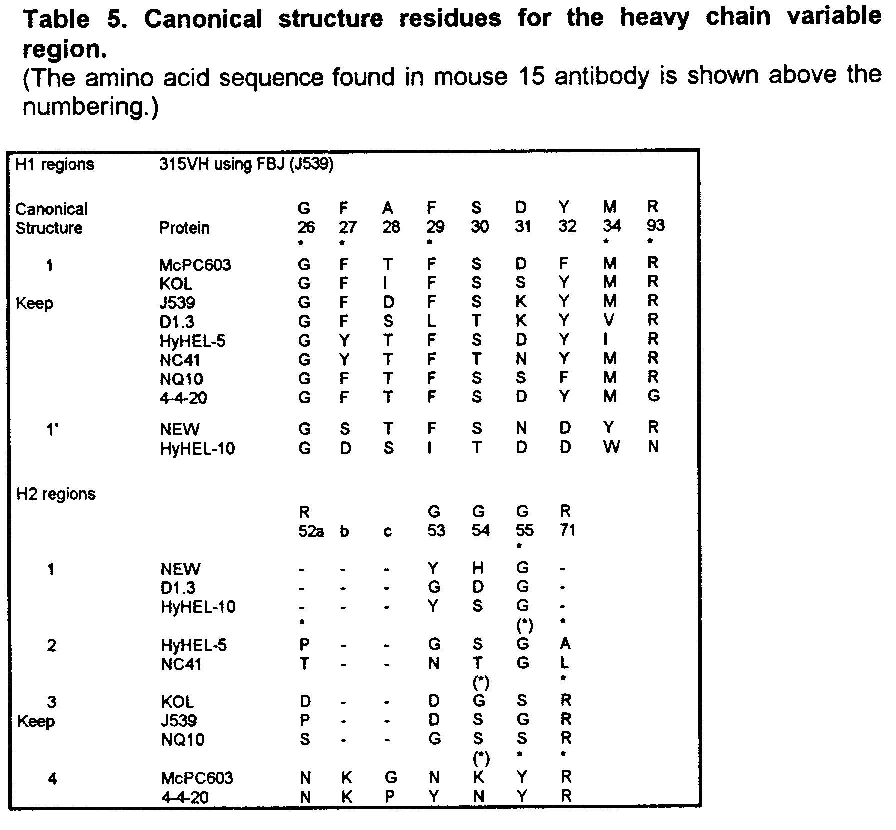

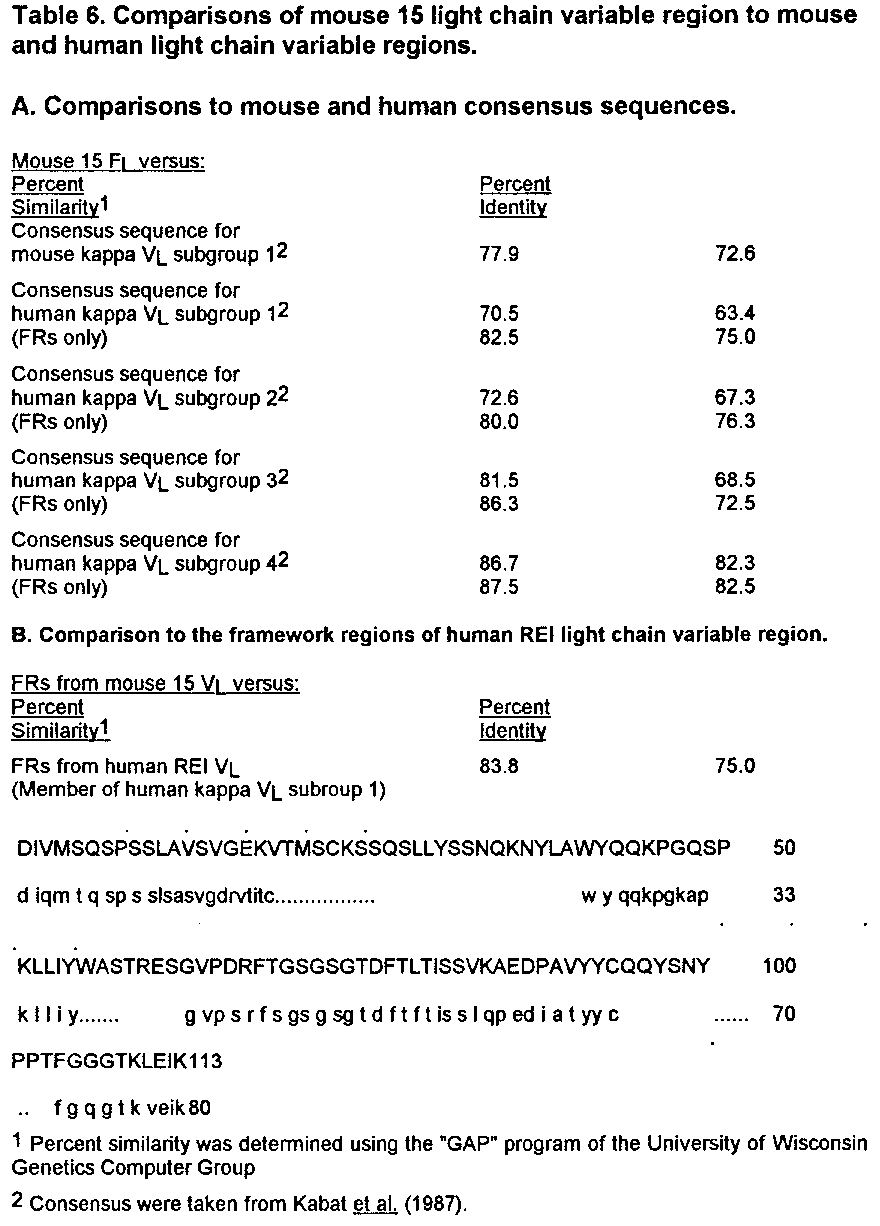

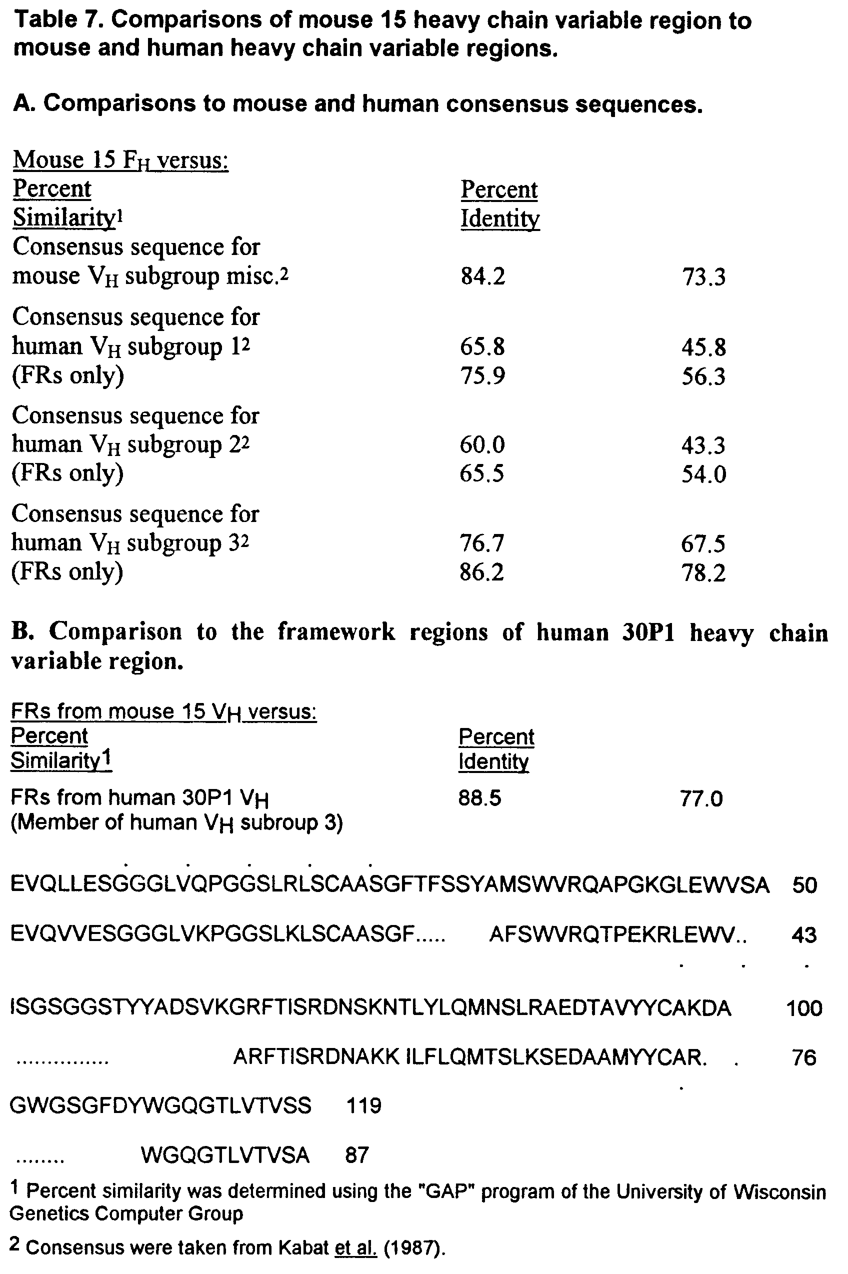

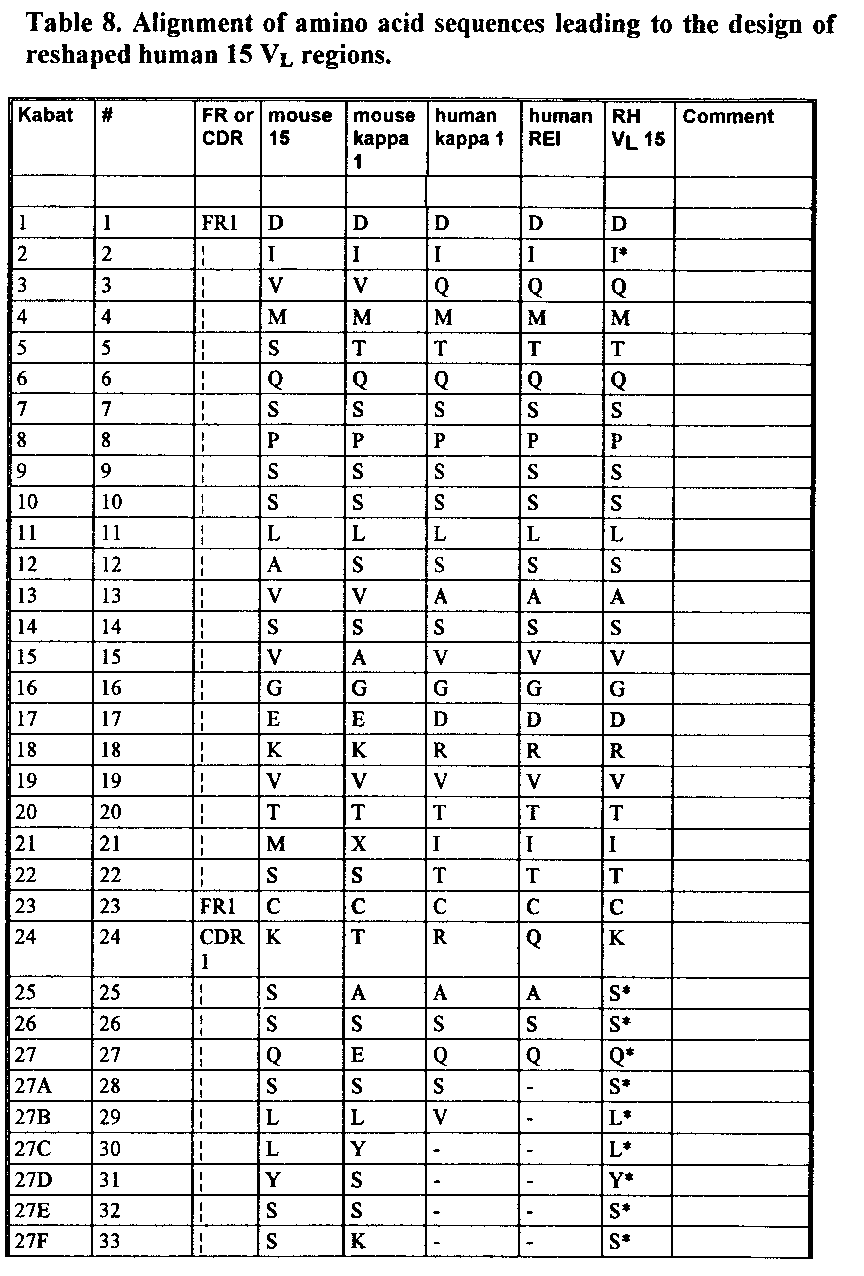

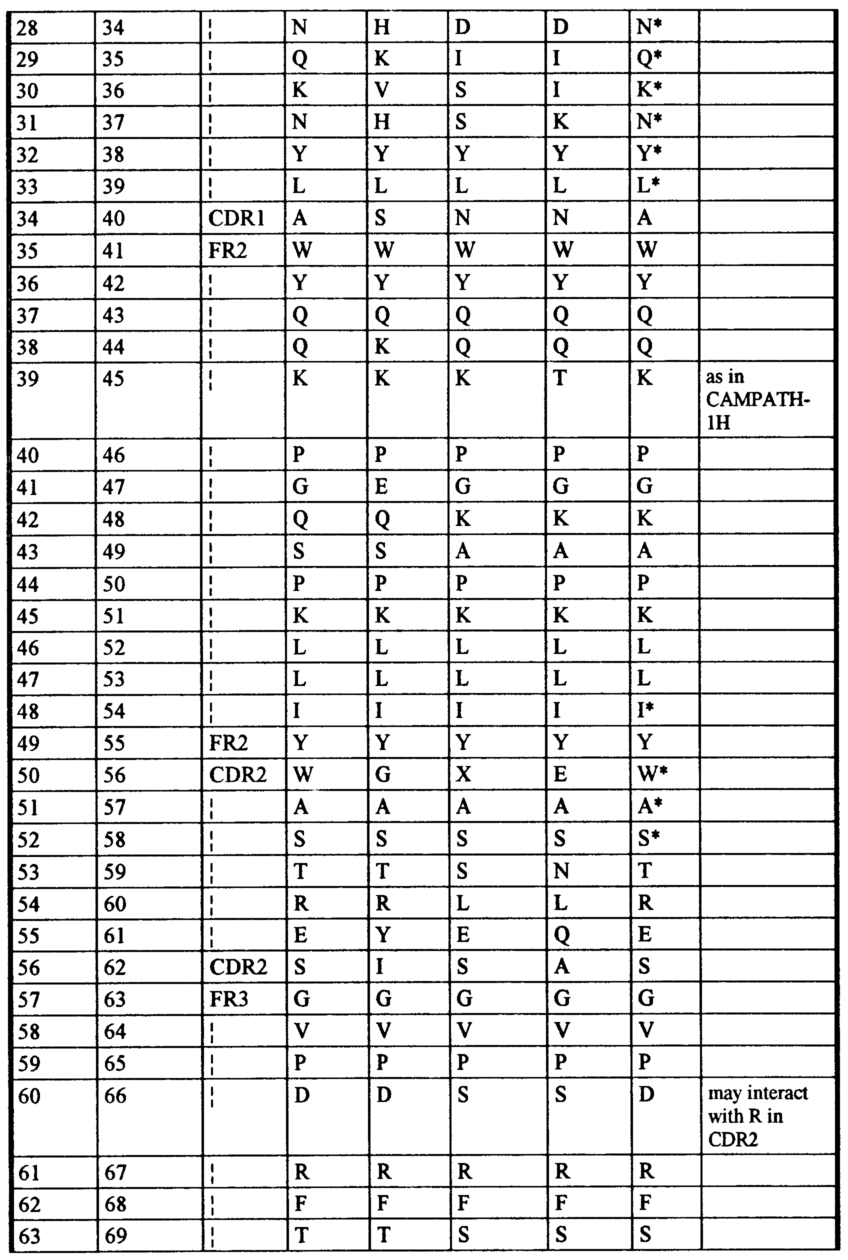

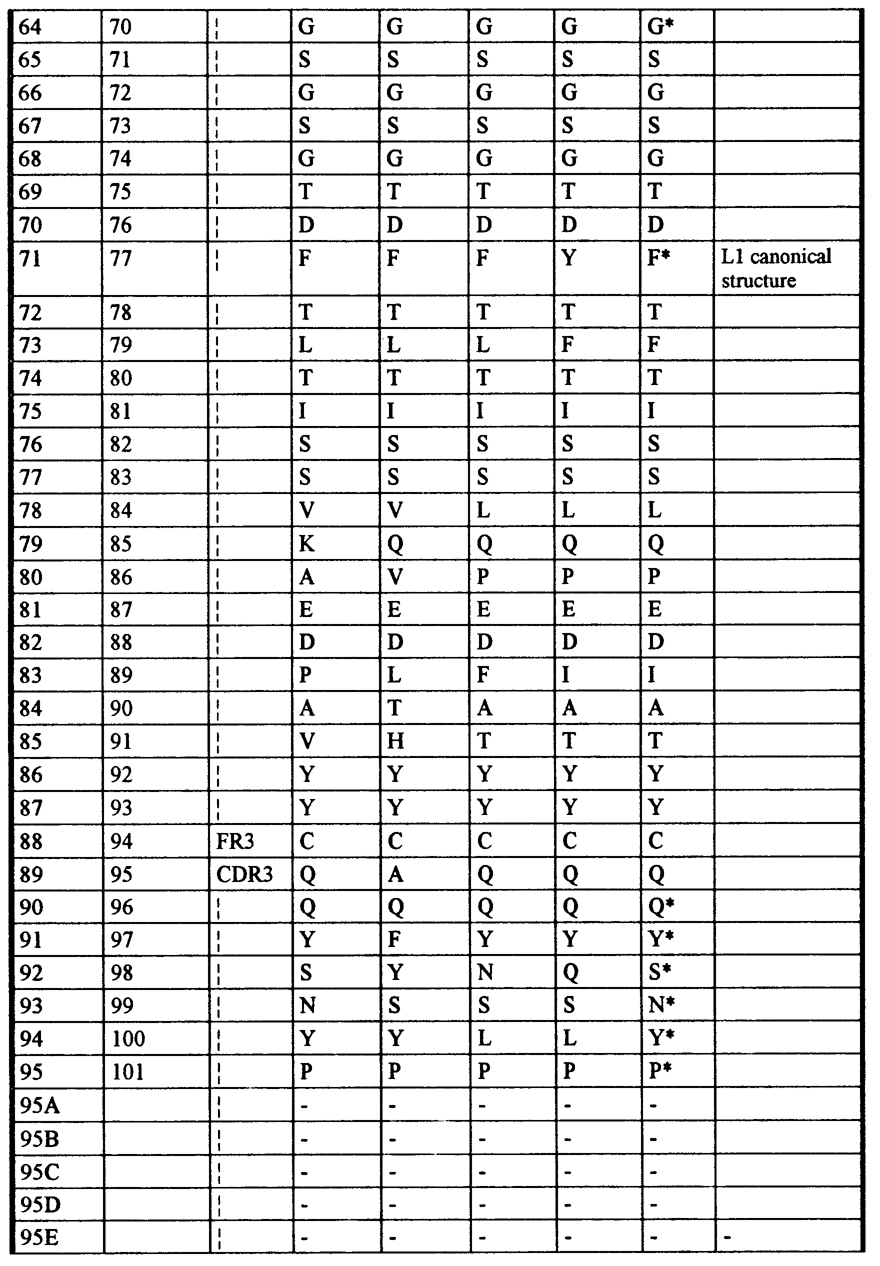

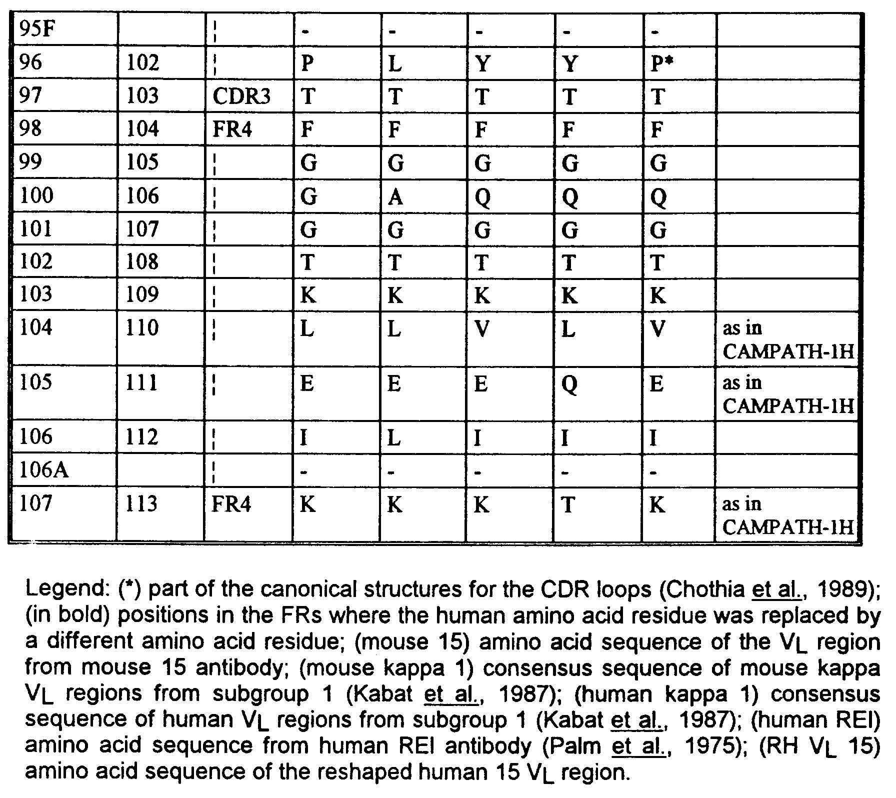

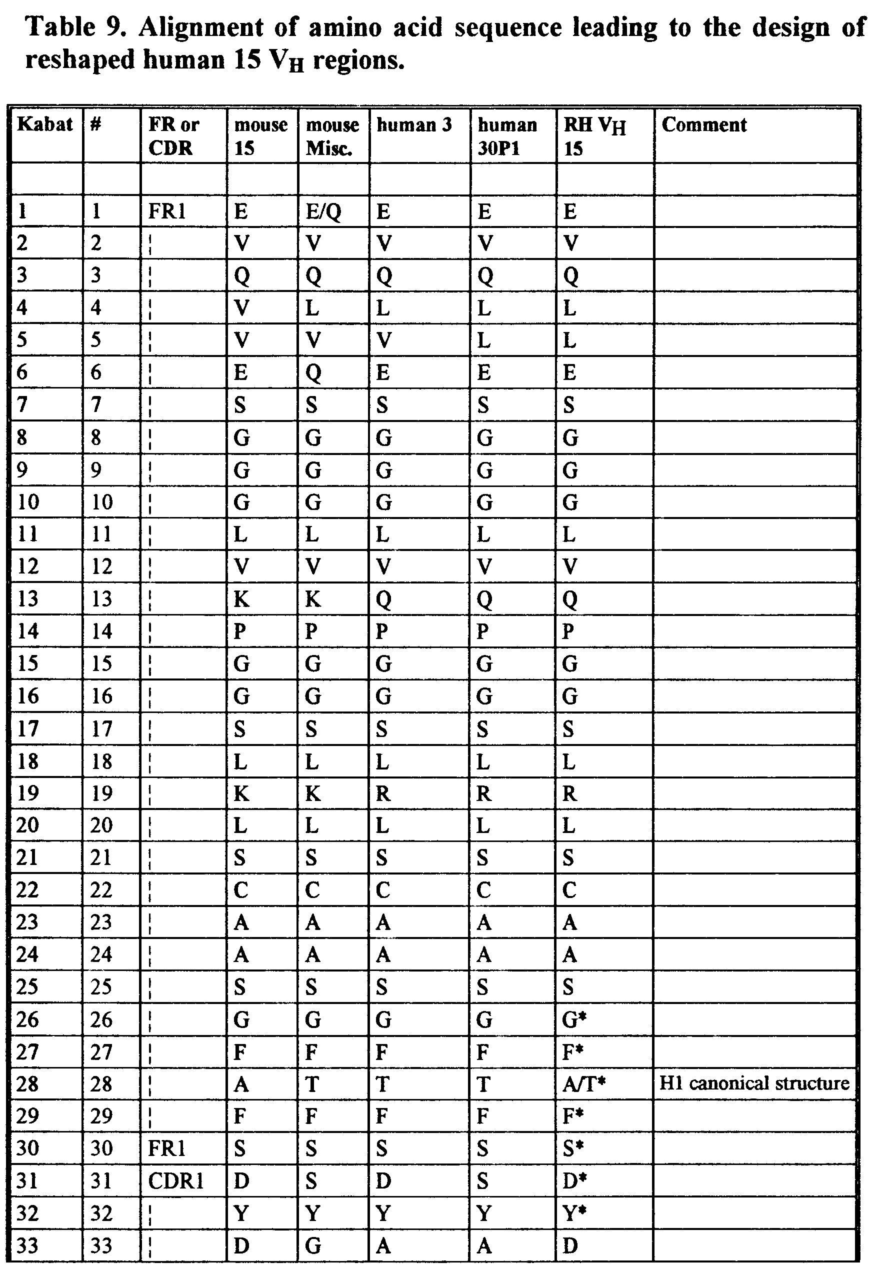

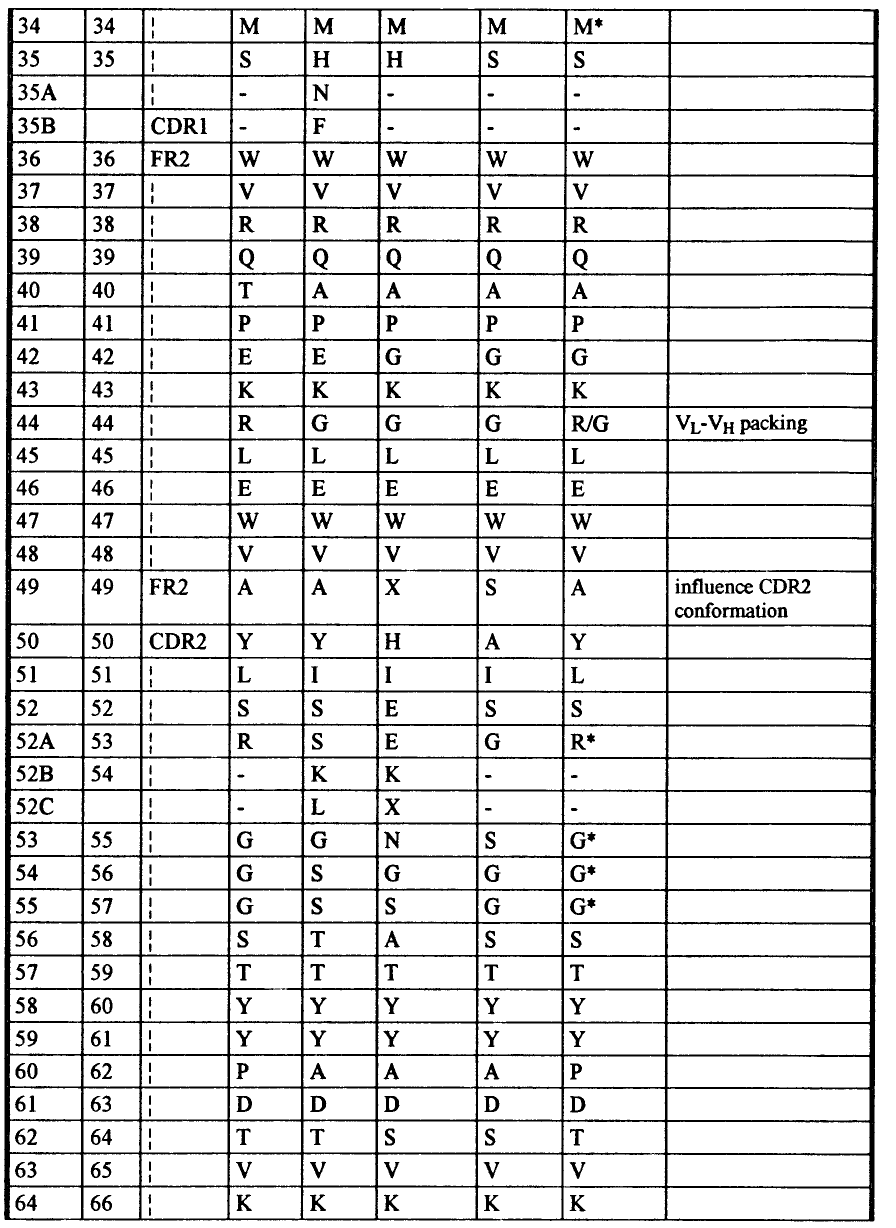

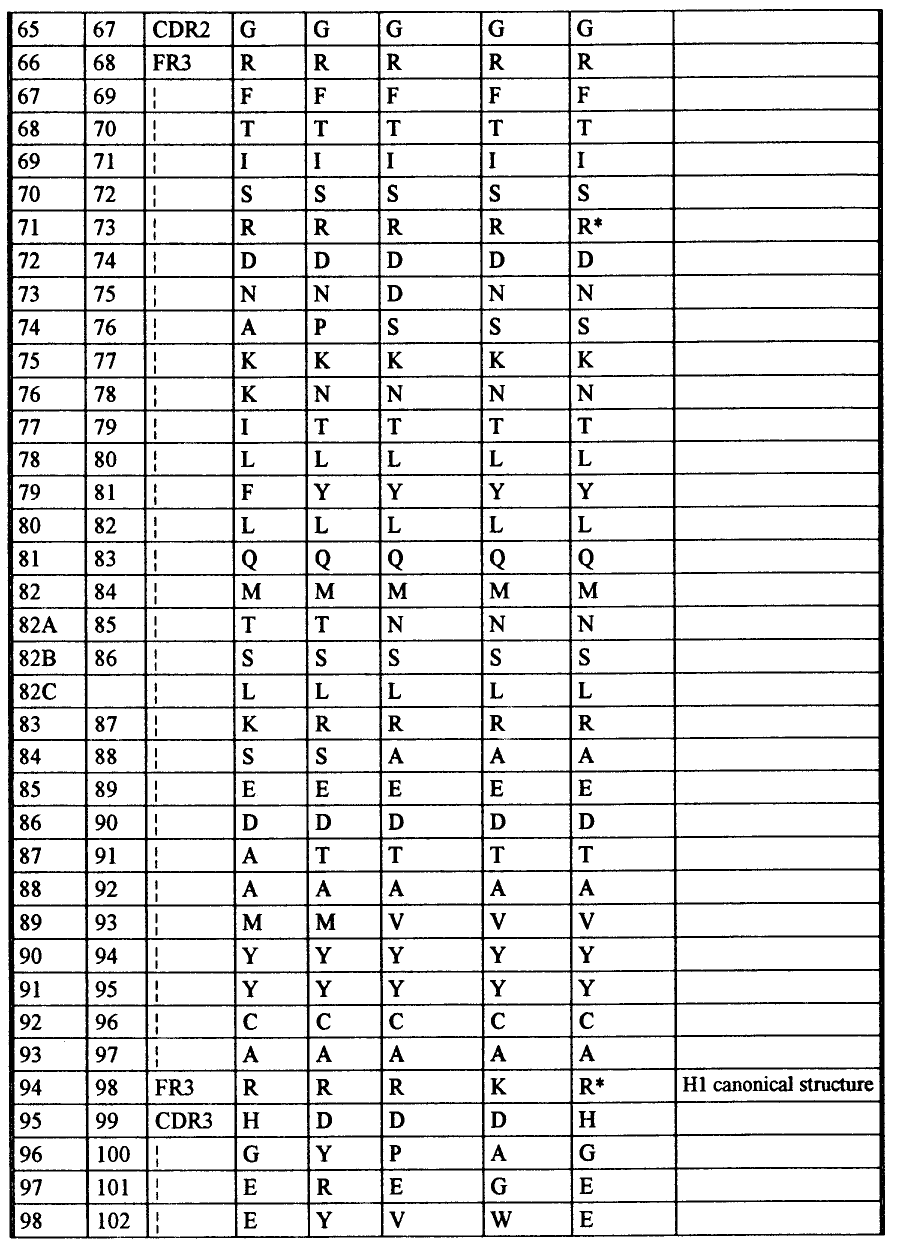

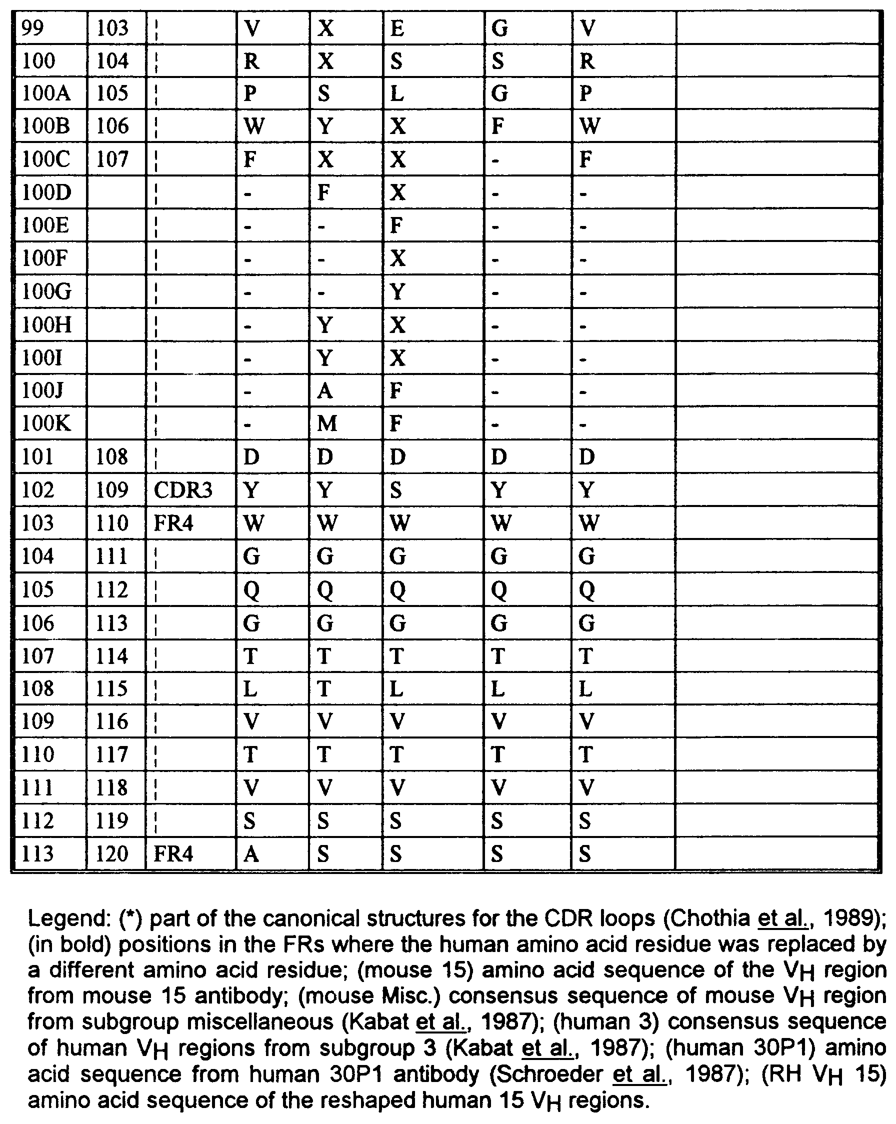

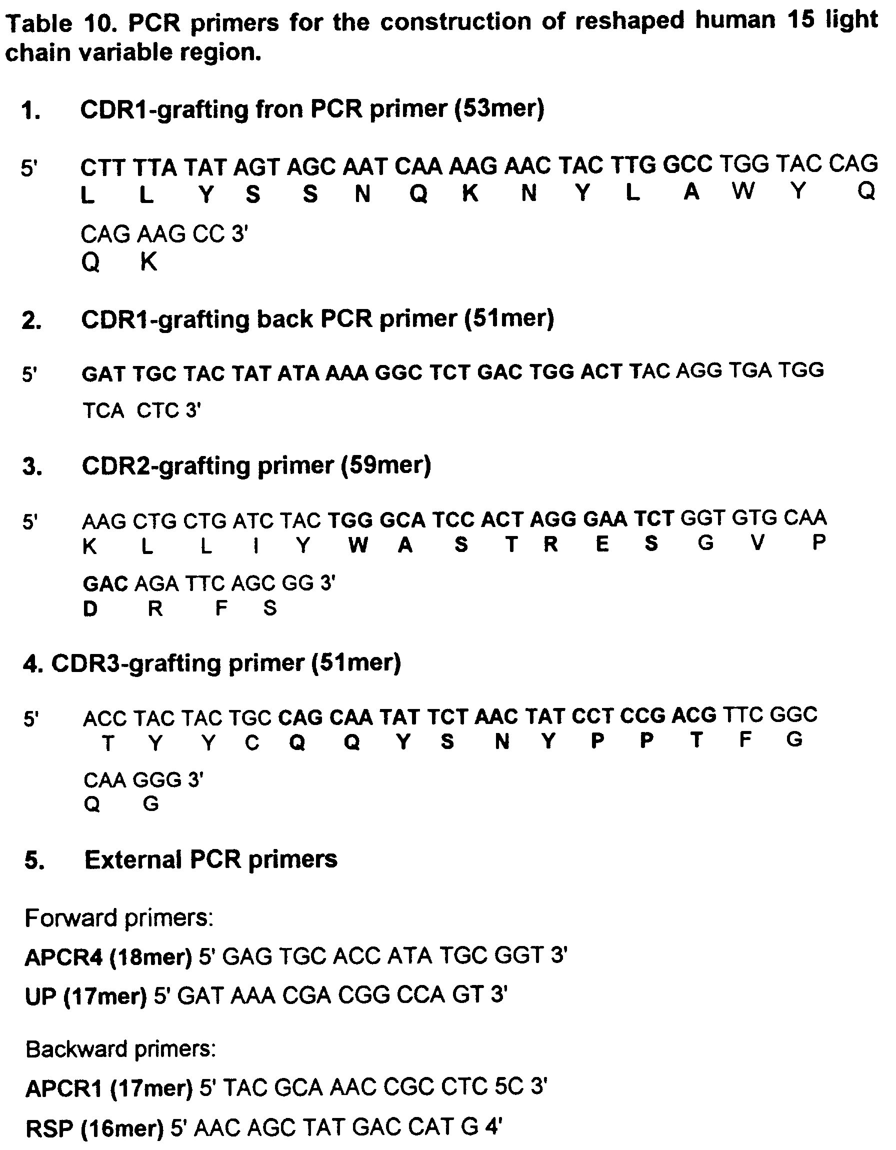

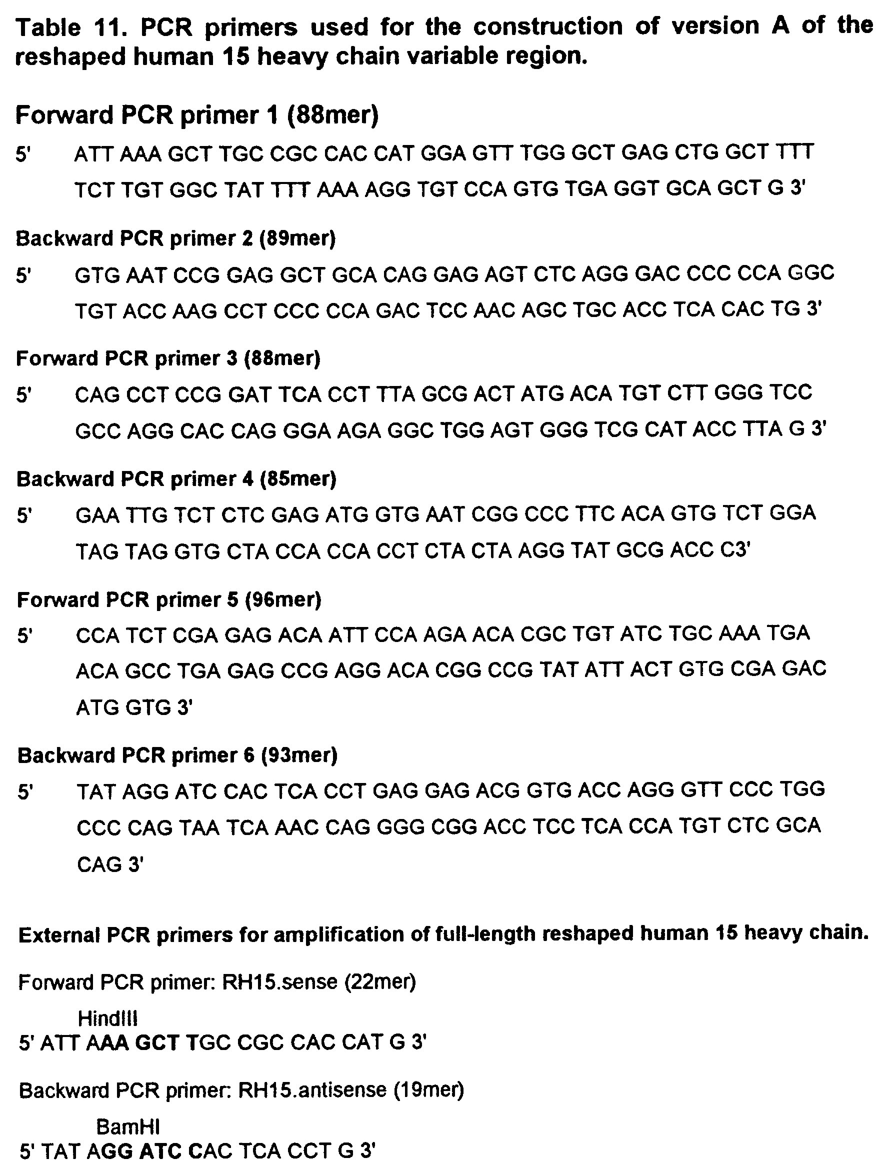

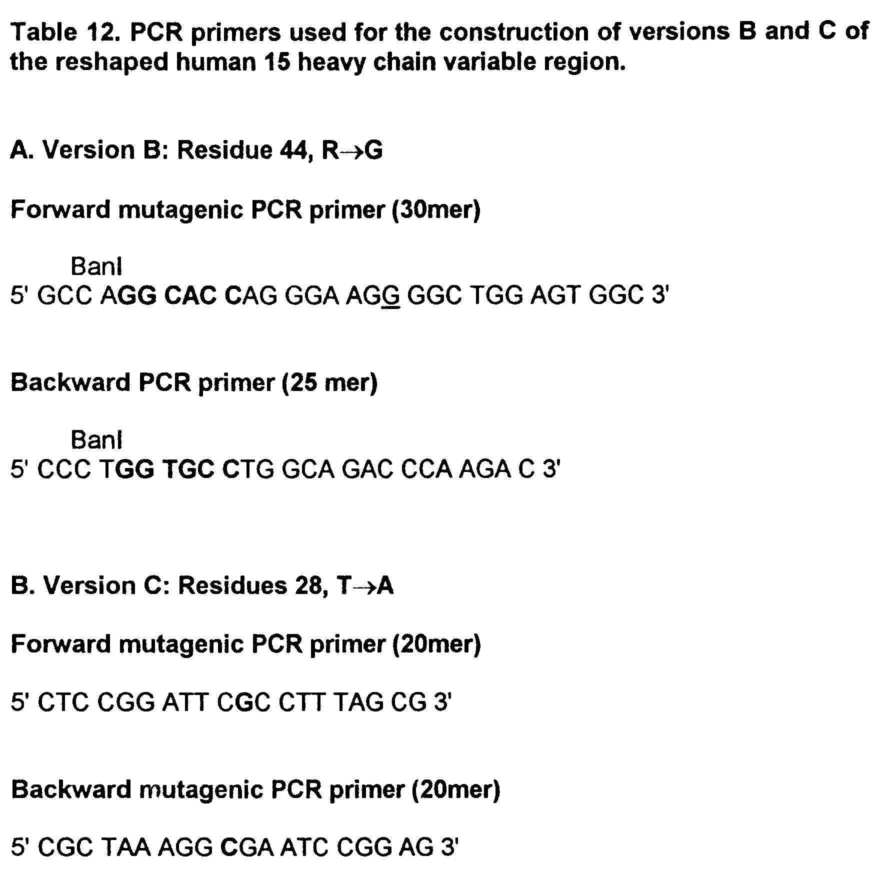

### EXAMPLES

### Example 1:

**PCR cloning and DNA sequencing of the mouse 15 variable regions.** Total RNA was isolated from MAb15-G1/K1-2 cells using a standard guanidinium isothiocyanate/cesium chloride method (Chirgwin et al., 1979). Cells (2×10⁸) were harvested and total RNA isolated. Poly(A)⁺ RNA was separated from total RNA using an oligo d(T) column (Qiagen). To ensure that the cells used for RNA isolation were producing functional MAb 15, the cell supernatants were collected and analyzed by ELISA. The supernatants were confirmed to obtain an antibody that bound to the MAb 15 target cells. First-strand cDNA synthesis was achieved directly from 2 µg of poly (A⁺) RNA essentially as described by Larrick et al. (1989) except that 25 u of human placental ribonuclease inhibitor, HRPI (Amersham plc, UK), was used in place of RNasin in each 10 µl reaction volume.

Mouse 15 light and heavy chain variable regions were PCR cloned using the product of the first-strand cDNA synthesis as a template DNA and the forward and reverse PCR primers designed by Jones and Bendig (1991). For PCR amplification of the mouse 15 heavy chain variable region, a 100 µl PCR reaction contained 10 mM Tris-HCl (pH 8.3), 50 mM potassium chloride, 1.5 mM MgCl₂, 200 µM dNTPs, 2.5 u Amplitaq (Perkin Elmer Cetus), 10 % of the first-strand cDNA synthesis reaction, 2.5 µM of the heavy chain gamma-1 constant region reverse primer, and 0.25 µM of each of the heavy chain leader sequence primers (groups 1 to 12). For PCR amplification of the mouse 15 light chain variable region, the PCR primers were changed to 3 µM of the kappa constant region reverse primer and 0.25 µM of each of the light chain leader sequence primers (groups 1 to 11). Each PCR reaction was overlayed with 50 µl of mineral oil and, after an initial melt at 94 °C for 1 min, cycled at 94 °C for 1 min, 50 °C for 1 min, and 72 °C for 1 min for 25 cycles with a final extension at 72 °C for 10 min, then cooled to 4 °C. The ramp time for each step was 30 sec, however, ramp times between primer annealing and extension were slower (2 min) to ensure that any mismatched primers would anneal properly. A sample of 10 µl of each PCR reaction was analyzed on an ethidium bromide-stained 1 % agarose gel. PCR products were purified using Qiagen PCR purification kits (Hybaid plc, UK) and then sequentially digested with Sa1I and XmaI restriction enyzmes. PCR fragments, after gel purifications, were ligated into pUC19 vectors and transformed into E. coli DH5α competent cells (Life Technologies). Positive clones were identified by restriction enzyme analysis and double-stranded DNA was sequenced using Sequenase version 2.0 kit (U.S. Biochemical Corporation).

### Example 2:

**Construction of chimeric 15 light and heavy chains.** The mouse 15 light and heavy chain variable regions were modified at the 5'- and 3'-ends to make them suitable for the construction of mouse-human chimeric light and heavy chains. The PCR primers used to adapt the mouse variable regions are shown in Table 3. Each PCR reaction contained as template DNA 10 ng of pUC19 plasmid containing the mouse light or heavy chain variable region, 10 mM Tris-HCl (pH 8.3), 50 mM KCl, 1.5 mM MgCl₂, 2.5 u Amplitaq, 200 µM dNTPs, and 1 µM of each PCR primer (forward and reverse). After an initial melt, 25 cycles of PCR were carried out at 94 °C for 1 min, 60 °C for 1 min, and 72 °C for 1 min, with a final extension at 72 °C for 10 min. PCR products were cloned into pUC19 vectors using HindIII and BamHI restriction enzyme sites, resequenced, and then subcloned into the HCMV expression vectors (Figure 3).

### Example 3:

**Cos cell transfections.** Cos cells were electroporated, using the Gene Pulsar apparatus (Biorad), with 10 µg each of the two HCMV expression vectors containing the DNA sequence coding for the chimeric light and heavy chains. The DNAs were added to 0.8 mls of a cos cell suspension in PBS (1 × 10⁷ cells/ml). A pulse was delivered at 1,900 volts, 25 µF capacitance. After a 10 min recovery period at room temperature, the cells were plated into 8 mls of DMEM (GIBCO) medium containing 10 % heat-inactivated, gamma globulin-free fetal calf serum. The cos cell supernatants were collected after 72 hrs incubation at 37 °C, centrifuged to remove any cell debris, and stored at 4 °C.

### Example 4:

**Protein A purification of chimeric and reshaped human 15 antibodies.** Antibody was purified from cos cell supernatants using 1 ml immobilized Protein A columns from an IgG purification kit (Pierce). Cos cell supernatants were filter-sterilized by passing them through a 0.2 µm filter. They were then mixed with an equal volume of binding buffer. The media was applied to a Protein A column e quilibrated with 5 mls of binding buffer. The column was washed with 15 mls of binding buffer and the antibody eluted with 8 mls of the elution buffer. The eluate was concentrated and the buffer changed to PBS/0.02 % sodium azide using a microconcentrator (Centricon 10, Amicon). The purified samples were then analyzed by ELISA to quantify the amount of human IgG present.

### Example 5:

**ELISA assay for human IgG.** Cos cell supernatants and purified antibody preparations were analyzed by ELISA for the amount of human IgG present. Goat anti-human IgG (whole molecule, Sigma) was used to coat 96 well plats at 0.25 µg/well. Cos cell supernatants, 100 µl/well, were added and incubated at 37 °C for 2 hrs. Bound antibody was detected using an alkaline phosphatase-conjugated goat anti-human IgG (gamma chain-specific, Sigma). After incubation and washing, the substrate buffer was added. The reaction was stopped using 1 M NaOH and the optical density at 405 nm was measured. Purified human IgG (Sigma) was used as a standard.

### Example 6:

**ELISA assay for antibody-binding to A431 cells.** The binding of purified antibody, and of antibody in cos cell supernatants, to A431 target cells was analyzed using a cell-based ELISA. Tissue culture 96 well microtiter plates (Nunclon, Delta SI) were coated, under sterile conditions, with 100 µg/ml of poly-D-lysine and incubated at room temperature (RT) for 15 min. The plates were then washed with sterile distilled water and dried at RT for 2 hrs. A suspension of A431 cells, 1 × 10⁸ cells/ml, was prepared in DMEM media, 100 µl/well was added, and the plated incubated at 37 °C overnight. Cells were washed with PBS and incubated at RT for 30 min in DMEM containing 1 % BSA and 0.1 M glycine. After washing, 200 µl of DMEM containing 1 % BSA was added per well and the plates were stored at -70 °C until required. The A431 cell-coated plates were washed with PBS containing 1 % BSA and 0.05 % Tween20 and then blocked overnight at 4 °C with PBS containing 2 % BSA and 0.05 % Tween20. Control mouse 15 antibody (1000 ng/ml) and the samples of antibody to be tested were added to the plates and further diluted 1:2 in wash buffer containing 10 % FCS. The plates were incubated at 37° for 90 min. Bound antibody was detected using either horseradish peroxidase-conjugated goat anti-mouse IgG and IgM antibody (Stratech-Jackson Immunochemicals) for bound mouse antibodies or horseradish peroxidase-conjugated goat anti-human IgG (gamma chain-specific) antibody (Sigma) to detect bound chimeric and reshaped human antibodies. After incubation at 37 °C for 90 min, the O-phenyl diamine (OPD) substrate was added, the plates incubated at room temperature for 20 min, and the reaction was stopped with 1 M H₂SO₄. Optical density was measured at 490 nm.

### Example 7:

**Construction of the reshaped human 15 light chain variable region.** The reshaped human 15 light chain variable region was constructed by grafting the CDRs from mouse 15 light chain variable region onto human REI human framework regions. A novel method based on PCR was used to carry out the CDR grafting. The PCR primers used are listed in Table 10 and the PCR reactions are illustrated in Figure 9.

The first PCR reaction contained 20 ng of template DNA (reshaped human 425 light chain variable region, version a, in a pUC18 vector), 0.5 µM each of APCR1 and CDR3 PCR primers, and 4 u of Amplitaq. The buffering conditions were as described above. Thermocycling was carried out for 25 cycles at 94 °C for 1 min, 37 °C for 2 min, and 72 °C for 1 min with a 5 min ramp time between annealing and extension. Product A (350 bps) was purified from an ethidium bromide-stained agarose gel and ethanol-precipitated. Double-stranded product A was then used as a reverse PCR primer in the second PCR reaction with 0.2 µM of CDR2 PCR primer and 20 ng of template DNA. Buffer and thermocycling conditions were as before. The 460 bp PCR product B was gel-purified and used as a PCR primer with 0.5 µM of CDR1 front PCR primer and 50 ng of DNA template. Again PCR product C was gel-purified. The fourth PCR reaction contained 20 ng of DNA template and 0.5 µM each of the external APCR4 primer and the CDR1back PCR primer. Thermocycling was carried out for 25 cycles at 94 °C for 1 min, 48 °C for 1 min, 72 °C for 1 min. PCR product D (270 bp) was obtained and gel purified. Double-stranded PCR products C and D were then joined in a PCR reaction containing equimolar amounts of each and cycled once at 94 °C for 2 min, 45 °C for 2 min, and 72 °C for 5 min with a 5 min ramp time between annealing and extension. External primers, universal primer (UP) and reverse sequencing primer (RSP), were then added at a final concentration of 0.5 µM each and cycled for 25 cycles at 94 °C for 1 min, 50 °C for 1 min, and 72 °C for 1 min. A full-length reshaped human 15 light chain variable region product (530 bp) was obtained, HindIII-BamHI digested, and subcloned into pUC19 vector.

### Example 8:

**Construction of the first version of reshaped human 15 heavy chain variable region.** The PCR method for assembly of a full-length heavy chain variable region gene is illustrated in Figure 11. Six overlapping synthetic oligonucleotides were designed with the assistance of the Stemloop program from the Sequence Analysis Software Package of Genetics Computer Group (University of Wisconsin, U.S.A.) to ensure that no secondary structures would be likely to form. The final designs of the DNA oligonucleotides were synthesised on a Milligen/Biosearch 7500 DNA synthesizer. These synthetic oligonucleotides ranged in length from 85 to 96 mers with approximately 19 bp overlaps between the oligonucleotides (Table 7, Figure 11). All six synthetic oligonucleotides were purified by PAGE in the presence of 8 M urea and 200 pmoles of each oligonucleotide was phosphorylated in a reaction containing 100 mM Tris-HCl (pH 8.0), 10 mM MgCl₂, 7 mM DTT, 2.5 mM ATP, and 20 u T4 polynucleotide kinase (New England Biolabs, U.S.A.). The PCR (90 µl volume) contained 5 pmoles of each kinased oligonucleotide (oligonucleotides 1 to 6). The buffering conditions were as described before. The reaction was cycled once at 95 °C for 1 min, 50 °C for 2 min, and 72 °C for 4 min with a 10 min ramp rate between and extension. Aliquots of 50 pmoles each of the forward and reverse external PCR primers were added and 20 PCR cycles done at 95 °C for 1 min, 60 °C for 1 min, and 72 °C for 2 min. The 450 bp full-length product was then HindIII-BamHI digested, gel-purified, and cloned into pUC18 vector.

### Example 9:

**Construction of versions B and C of reshaped 15 heavy chain variable region.** Reshaped human 15 heavy chain variable region versions B and C were constructed using the PCR primers listed in Table 12. The PCR reactions are illustrated in Figure 13. In the construction of version C, the two primary PCR reactions contained 20 ng of reshaped human 15 heavy chain variable region (version B) as template DNA and 0.5 µM of each primer. The reaction was thermocycled for 25 cycles at 94 °C for 1 min, 45 °C for 1 min, and 72 °C for 1 min. The PCR products were gel-purified. The two PCR products were joined together in a PCR reaction that contained 0.5 pmoles of each product. The reaction was cycled once at 94 °C for 2 min, 50 °C for 2 min, and 72 °C for 5 min with ramp times between annealing and extension of 5 min. Following this one cycle, 0.5 µM of each external primer was added and 25 further cycles at 94 °C for 1 min, 50 °C for 1 min, and 72 °C for 1 min were carried out. Version B was constructed as described for version C except that the template DNA was reshaped human 15 heavy chain variable region (version A) and the two products from the primary PCR reactions were joined together using the unique BanI restriction enzyme sites present in the reshaped human 15 heavy chain variable region gene.

## Claims

1. Humanized reshaped monoclonal antibody derived from murine monoclonal antibody MAb 15 (DSM ACC2117).

2. Humanized reshaped monoclonal antibody according to claim 1 having the amino acid and DNA sequence of the variable regions indicated in Fig. 10 and 12.

3. Humanized reshaped monoclonal antibody according to claim 2 having the amino acid and DNA sequence of the light chain variable region of Fig. 10.

4. Humanized reshaped monoclonal antibody according to claim 2 having the amino acid and DNA sequence of the heavy chain variable region of Fig. 12.

5. Amino acid and DNA sequence of Figure 10.

6. The CDR regions of claim 5.

7. Amino acid and DNA sequence of Figure 12.

8. The CDR regions of claim 7.

9. Process for the preparation of a monoclonal antibody according to claim 1 - 4 by cultivating transformed host cells in a culture medium and purification and isolation the expressed antibody proteins according to standard techniques comprising the following steps:
(i) cloning and sequencing the murine MAb 15 light and heavy variable regions,
(ii) constructing, expressing and analyzing chimeric MAb 15 antibody,
(iii) modelling the structure of murine MAb 15 variable regions,
(iv) designing reshaped human MAb 15 variable regions,
(v) synthesizing, partially synthesizing or isolating an oligonucleotide sequence which codes for the amino acid sequence of constant regions of the light and heavy chain of a human immunoglobulin, and
(vi) constructing, expressing and analyzing the complete reshaped human MAb 15 antibodies.

10. Pharmaceutical composition comprising a monoclonal antibody of claim 1 - 4, optionally together with a pharmaceutically acceptable carrier.

11. Pharmaceutical composition of claim 10 for use in the treatment of tumors.

12. Pharmaceutical composition of claim 11 for use in the treatment of lung cancer.

13. Use of a monoclonal antibody of claim 1 - 4 for the manufacture of a drug related to tumors, especially lung cancer.

14. DNA- and amino acid sequence of Fig. 1, from which the sequences according to claims 5 - 8 derive.
